# EUROPEAN PATENT APPLICATION

(11) **EP 2 468 304 A2**
(43) Date of publication of application: **27.06.2012**
(21) Application number: 12160182.7
(22) Date of filing: 10.02.2006
(51) Int. Cl.: A61K 47/48

(54) **Process for preparing stable drug conjugates**

(30) Priority: 11.02.2005 US 652434 P
(62) Divisional of application: 06720665.6
(71) Applicant: ImmunoGen, Inc., Waltham, MA 02451 (US)
(72) Inventor: Chari, Ravi, Newton, MA 02461 (US); Zhang, Wei, Chesterbrook, PA 19087 (US); Meshulam, Deborah H., Brookline, MA 02446 (US); Dai, Yong, Northborough, MA 01532 (US); Wang, Yong, Wayland, MA 01778 (US); Amphlett, Godfrey W., Cambridge, MA 02138 (US)
(74) Representative: Pfenning, Meinig & Partner GbR

(57) **Abstract**

The invention provides a process for preparing a conjugate comprising a cell binding agent chemically coupled to a drug. The process comprises covalently attaching a linker to a cell binding agent, which then is conjugated to a drug, as well as purification and holding steps, and optionally a tangential flow filtration (TFF) step.

## Description

### FIELD OF THE INVENTION

This invention pertains to a method for preparing a conjugate comprising a cell binding agent chemically coupled to a drug.

### BACKGROUND OF THE INVENTION

The treatment of cancer has progressed significantly with the development of pharmaceuticals that more efficiently target and kill cancer cells. To this end, researchers have taken advantage of cell-surface receptors and antigens selectively expressed by cancer cells to develop drugs based on antibodies that bind the tumor-specific or tumor-associated antigens. In this regard, cytotoxic molecules such as bacteria and plant toxins, radionuclides, and certain chemotherapeutic drugs have been chemically linked to monoclonal antibodies that bind tumor-specific or tumor-associated cell surface antigens (see, e.g., International (PCT) Patent Application Publications WO 00/02587, WO 02/060955, and WO 02/092127, U.S. Patents 5,475,092, 6,340,701, and 6,171,586, U.S. Patent Application Publication 2003/0004210 A1, and Ghetie et al., J. Immunol. Methods, 112, 267-277 (1988)). Such compounds are typically referred to as toxin, radionuclide, and drug "conjugates," respectively. Often they also are referred to as immunoconjugates, radioimmunoconjugates, and immunotoxins. Tumor cell killing occurs upon binding of the drug conjugate to a tumor cell and activation of the cytotoxic activity of the maytansinoid. The selectivity afforded by drug conjugates minimizes toxicity to normal cells, thereby enhancing tolerability of the drug in the patient.

Processes for conjugating antibodies to sulfhydryl-containing cytotoxic agents such as maytansinoids have been described previously (see, e.g., U.S. Patents 5,208,020, 5,416,064, and 6,441,163. For example, U.S. Patents 5,208,020 and 5,416,064 disclose a process for manufacturing antibody-maytansinoid conjugates wherein the antibody is first modified with a heterobifunctional reagent such as described in U.S. Patents 4,149,003 and 4,563,304. U.S. Patents 5,208,020 and 5,416,064 further describe conjugation of a modified antibody with an excess of a sulfhydryl-containing cytotoxic agent at pH 7, followed by purification on SEPHADEX™ G25 chromatography columns. Purification of antibody-drug conjugates by size exclusion chromatography (SEC) also has been described (see, e.g., Liu et al., Proc. Natl. Acad. Sci. (USA), 93, 8618-8623 (1996), and Chari et al., Cancer Research, 52, 127-131 (1992)).

International Patent Application Publication WO 03/057163 describes modifying an antibody at pH 5.0-8.0, removing unreacted linker by tangential flow filtration (TFF), conjugating the antibody at pH 6.0-6.5 in dimethylacetamide (DMA) and/or acetonitrile, and purifying the conjugate via ion exchange chromatography (e.g., ceramic hydroxyapatite (CHT)). The antibody is eluted with an increased concentration of sodium chloride. Alternatively, U.S. Patent 6,441,163 describes performing antibody-drug conjugation in a single step using a preformed linker-drug compound.

Despite the advances in preparing antibody-drug conjugates, current methods are limited by several factors. For example, the binding of a bifunctional cross-linking agent to an antibody is heterogeneous under the conditions currently employed in the art, leading to the slow release of the drug from the conjugate and conjugate instability. Moreover, some protein-2-pyridyl disulphide conjugate derivatives generated with hydroxysuccimide esters are unstable and decay slowly. Another limitation is that the conjugation process itself leads to the formation of undesirable degradation products, such as high and low molecular weight species of conjugate and precipitates, which can result in lower process yields.

Thus, in view of the above, there remains a need for improved methods of preparing drug conjugate compositions that are more stable and are of higher purity than currently available drug conjugate compositions. The invention provides such a method. These and other advantages of the invention, as well as additional inventive features, will be apparent from the description of the invention provided herein.

### BRIEF SUMMARY OF THE INVENTION

The invention provides a novel process for preparing a conjugate comprising a cell binding agent chemically coupled to a drug. The process comprises (a) modifying the cell binding agent with a bifunctional crosslinking reagent to covalently attach a linker to the cell binding agent and thereby prepare a first mixture comprising cell binding agents having linkers stably and unstably bound thereto, (b) subjecting the first mixture to non-adsorptive chromatography to purify the cell binding agents having linkers bound thereto from other components of the first mixture and thereby prepare a purified first mixture, (c) conjugating a drug to the cell binding agents having linkers bound thereto by reacting the cell binding agents having linkers bound thereto with the drug in a solution having a pH of about 4.5 to about 8 to prepare a second mixture comprising (i) cell binding agent chemically coupled through the linker to the drug (ii) free drug, and (iii) solvents and reaction by products (d) subjecting the second mixture to non-adsorptive chromatography to purify the cell binding agents chemically coupled through the linkers to the drug from the other components of the second mixture and thereby prepare a purified second mixture, (e) optionally subjecting the purified second mixture to tangential flow filtration (TFF) or absorptive chromatography to isolate a conjugate comprising the cell binding agent chemically coupled to the drug, and (f) holding the mixture between at least one of steps a-b, steps b-c, steps c-d, and steps d-e to release the unstably bound linkers from the cell binding agent.

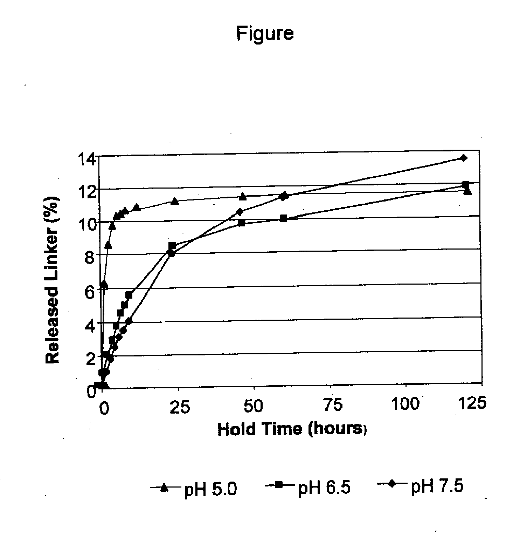

### BRIEF DESCRIPTION OF THE SEVERAL VIEWS OF THE DRAWING(S)

The Figure is a graph of the release of 4-(2-pyridyldithio)pentanoic acid (PPA) linker (% of total linker originally bound) from huN901 modified with N-succinimidyl 4-(2-pyridyldithio)pentanoate (SPP) after holding at various pH levels.

### DETAILED DESCRIPTION OF THE INVENTION

The invention provides a process for preparing compositions of stable conjugates comprising a cell binding agent chemically coupled to a drug, wherein the compositions are substantially free of unstable conjugates. Such compositions can be used for treating diseases because of the stability and high purity of the conjugates. Compositions comprising a cell binding agent, such as an antibody, chemically coupled to a drug, such as a maytansinoid, are described in, for example, U.S. Patent Application Publication 2004/0241174 A1.

One of ordinary skill in the art will appreciate that conjugates comprising an antibody chemically coupled to a drug ("antibody-drug conjugates") typically are prepared by purifying an antibody, modifying the purified antibody followed by single step purification, conjugation, and a final purification step. The present invention improves upon such methods by including a hold step, as well as desirably diafiltration, achieved by a membrane-based Tangential Flow Filtration (TFF) process, after the final purification step. In addition, the invention improves upon previous methods by modifying the pH during the conjugation reaction, such that drug utilization, process yields, reduction of side reactions, and the purity of the cell binding agent-drug conjugate are optimized. Optionally, the addition of sucrose during the process has been shown to increase process yields.

In this respect, the inventive process comprises (a) modifying the cell binding agent with a bifunctional crosslinking reagent to covalently attach a linker to the cell binding agent and thereby prepare a first mixture comprising cell binding agents having linkers stably and unstably bound thereto, (b) subjecting the first mixture to non-adsorptive chromatography to purify the cell binding agents having linkers bound thereto from other components of the first mixture and thereby prepare a purified first mixture, (c) conjugating a drug to the cell binding agents having linkers bound thereto by reacting the cell binding agents having linkers bound thereto with the drug in a solution having a pH of about 4.5 to about 8 to prepare a second mixture comprising (i) cell binding agent chemically coupled through the linker to the drug, (ii) free drug, and (iii) solvents and reaction by products (d) subjecting the second mixture to non-adsorptive chromatography to purify the cell binding agents chemically coupled through the linkers to the drug from the other components of the second mixture and thereby prepare a purified second mixture, (e) optionally subjecting the purified second mixture to tangential flow filtration (TFF) or absorptive chromatography to isolate a conjugate comprising the cell binding agent chemically coupled to the drug, and (f) holding the mixture between at least one of steps a-b, steps b-c, steps c-d, and steps d-e to release the unstably bound linkers from the cell binding agent.

The cell binding agent can be any suitable agent that binds to a cell, typically and preferably an animal cell (e.g., a human cell). The cell binding agent preferably is a peptide or a polypeptide. Suitable cell binding agents include, for example, antibodies (e.g., monoclonal antibodies and fragments thereof), lymphokines, hormones, growth factors, nutrient-transport molecules (e.g., transferrin), and any other agent or molecule that specifically binds a target molecule on the surface of a cell.

The term "antibody," as used herein, refers to any immunoglobulin, any immunoglobulin fragment, such as Fab, F(ab')₂, dsFv, sFv, diabodies, and triabodies, or immunoglobulin chimera, which can bind to an antigen on the surface of a cell (e.g., which contains a complementarity determining region (CDR)). Any suitable antibody can be used as the cell binding agent. One of ordinary skill in the art will appreciate that the selection of an appropriate antibody will depend upon the cell population to be targeted. In this regard, the type and number of cell surface molecules (i.e., antigens) that are selectively expressed in a particular cell population (typically and preferably a diseased cell population) will govern the selection of an appropriate antibody for use in the inventive composition. Cell surface expression profiles are known for a wide variety of cell types, including tumor cell types, or, if unknown, can be determined using routine molecular biology and histochemistry techniques.

The antibody can be polyclonal or monoclonal, but is most preferably a monoclonal antibody. As used herein, "polyclonal" antibodies refer to heterogeneous populations of antibody, typically contained in the sera of immunized animals. "Monoclonal" antibodies refer to homogenous populations of antibody molecules that are specific to a particular antigen. Monoclonal antibodies are typically produced by a single clone of B lymphocytes ("B cells"). Monoclonal antibodies may be obtained using a variety of techniques known to those skilled in the art, including standard hybridoma technology (see, e.g., Köhler and Milstein, Eur. J. Immunol., 5, 511-519 (1976), Harlow and Lane (eds.), Antibodies: A Laboratory Manual, CSH Press (1988), and C.A. Janeway et al. (eds.), Immunobiology, 5th Ed., Garland Publishing, New York, NY (2001)). In brief, the hybridoma method of producing monoclonal antibodies typically involves injecting any suitable animal, typically and preferably a mouse, with an antigen (i.e., an "immunogen"). The animal is subsequently sacrificed, and B cells isolated from its spleen are fused with human myeloma cells. A hybrid cell is produced (i.e., a "hybridoma"), which proliferates indefinitely and continuously secretes high titers of an antibody with the desired specificity *in vitro.* Any appropriate method known in the art can be used to identify hybridoma cells that produce an antibody with the desired specificity. Such methods include, for example, enzyme-linked immunosorbent assay (ELISA), Western blot analysis, and radioimmunoassay. The population of hybridomas is screened to isolate individual clones, each of which secretes a single antibody species to the antigen. Because each hybridoma is a clone derived from fusion with a single B cell, all the antibody molecules it produces are identical in structure, including their antigen binding site and isotype. Monoclonal antibodies also may be generated using other suitable techniques including EBV-hybridoma technology (see, e.g., Haskard and Archer, J. Immunol. Methods, 74(2), 361-67 (1984), and Roder et al., Methods Enzymol., 121, 140-67 (1986)), bacteriophage vector expression systems (see, e.g., Huse et al., Science, 246, 1275-81 (1989)), or phage display libraries comprising antibody fragments, such as Fab and scFv (single chain variable region) (see, e.g., U.S. Patents 5,885,793 and 5,969,108, and International Patent Application Publications WO 92/01047 and WO 99/06587).

The monoclonal antibody can be isolated from or produced in any suitable animal, but is preferably produced in a mammal, more preferably a mouse, and most preferably a human. Methods for producing an antibody in mice are well known to those skilled in the art and are described herein. With respect to human antibodies, one of ordinary skill in the art will appreciate that polyclonal antibodies can be isolated from the sera of human subjects vaccinated or immunized with an appropriate antigen. Alternatively, human antibodies can be generated by adapting known techniques for producing human antibodies in non-human animals such as mice (see, e.g., U.S. Patents 5,545,806, 5,569,825, and 5,714,352, and U.S. Patent Application Publication 2002/0197266 A1).

While being the ideal choice for therapeutic applications in humans, human antibodies, particularly human monoclonal antibodies, typically are more difficult to generate than mouse monoclonal antibodies. Mouse monoclonal antibodies, however, induce a rapid host antibody response when administered to humans, which can reduce the therapeutic or diagnostic potential of the antibody-drug conjugate. To circumvent these complications, a monoclonal antibody preferably is not recognized as "foreign" by the human immune system. To this end, phage display can be used to generate the antibody. In this regard, phage libraries encoding antigen-binding variable (V) domains of antibodies can be generated using standard molecular biology and recombinant DNA techniques (see, e.g., Sambrook et al. (eds.), Molecular Cloning, A Laboratory Manual, 3rd Edition, Cold Spring Harbor Laboratory Press, New York (2001)). Phage encoding a variable region with the desired specificity are selected for specific binding to the desired antigen, and a complete human antibody is reconstituted comprising the selected variable domain. Nucleic acid sequences encoding the reconstituted antibody are introduced into a suitable cell line, such as a myeloma cell used for hybridoma production, such that human antibodies having the characteristics of monoclonal antibodies are secreted by the cell (see, e.g., Janeway et al., *supra*, Huse et al., *supra,* and U.S. Patent 6,265,150). Alternatively, monoclonal antibodies can be generated from mice that are transgenic for specific human heavy and light chain immunoglobulin genes. Such methods are known in the art and described in, for example U.S. Patents 5,545,806 and 5,569,825, and Janeway et al., *supra.* Most preferably the antibody is a humanized antibody. As used herein, a "humanized" antibody is one in which the complementarity-determining regions (CDR) of a mouse monoclonal antibody, which form the antigen binding loops of the antibody, are grafted onto the framework of a human antibody molecule. Owing to the similarity of the frameworks of mouse and human antibodies, it is generally accepted in the art that this approach produces a monoclonal antibody that is antigenically identical to a human antibody but binds the same antigen as the mouse monoclonal antibody from which the CDR sequences were derived. Methods for generating humanized antibodies are well known in the art and are described in detail in, for example, Janeway et al., *supra,* U.S. Patents 5,225,539, 5,585,089 and 5,693,761, European Patent 0239400 B1, and United Kingdom Patent 2188638. Humanized antibodies can also be generated using the antibody resurfacing technology described in U.S. Patent 5,639,641 and Pedersen et al., J. Mol. Biol., 235, 959-973 (1994). While the antibody employed in the conjugate of the inventive composition most preferably is a humanized monoclonal antibody, a human monoclonal antibody or a mouse monoclonal antibody, as described above, are also within the scope of the invention.

Antibody fragments that have at least one antigen binding site, and thus recognize and bind to at least one antigen or receptor present on the surface of a target cell, also are within the scope of the invention. In this respect, proteolytic cleavage of an intact antibody molecule can produce a variety of antibody fragments that retain the ability to recognize and bind antigens. For example, limited digestion of an antibody molecule with the protease papain typically produces three fragments, two of which are identical and are referred to as the Fab fragments, as they retain the antigen binding activity of the parent antibody molecule. Cleavage of an antibody molecule with the enzyme pepsin normally produces two antibody fragments, one of which retains both antigen-binding arms of the antibody molecule, and is thus referred to as the F(ab')₂ fragment. Reduction of an F(ab')₂ fragment with dithiothreitol or mercaptoethylamine produces a fragment referred to as an Fab' fragment. A single-chain variable region fragment (sFv) antibody fragment, which consists of a truncated Fab fragment comprising the variable (V) domain of an antibody heavy chain linked to a V domain of a light antibody chain via a synthetic peptide, can be generated using routine recombinant DNA technology techniques (see, e.g., Janeway et al., *supra*). Similarly, disulfide-stabilized variable region fragments (dsFv) can be prepared by recombinant DNA technology (see, e.g., Reiter et al., Protein Engineering, 7, 697-704 (1994)). Antibody fragments of the present invention, however, are not limited to these exemplary types of antibody fragments. Any suitable antibody fragment that recognizes and binds to a desired cell surface receptor or antigen can be employed. Antibody fragments are further described in, for example, Parham, J. Immunol., 1312895-2902 (1983), Spring et al., J. Immunol., 113, 470-478 (1974), and Nisonoff et al., Arch. Biochem. Biophys., 89, 230-244 (1960). Antibody-antigen binding can be assayed using any suitable method known in the art, such as, for example, radioimmunoassay (RIA), ELISA, Western blot, immunoprecipitation, and competitive inhibition assays (see, e.g., Janeway et al., *supra,* and U.S. Patent Application Publication 2002/0197266 A1).

In addition, the antibody can be a chimeric antibody or antigen binding fragments thereof. By "chimeric" is meant that the antibody comprises at least two immunoglobulins, or fragments thereof, obtained or derived from at least two different species (e.g., two different immunoglobulins, such as a human immunoglobulin constant region combined with a murine immunoglobulin variable region). The antibody also can be a domain antibody (dAb) or an antigen binding fragment thereof, such as, for example, a camelid antibody (see, e.g., Desmyter et al., Nature Struct. Biol., 3, 752, (1996)), or a shark antibody, such as, for example, a new antigen receptor (IgNAR) (see, e.g., Greenberg et al., Nature, 374, 168 (1995), and Stanfield et al., Science, 305, 1770-1773 (2004)).

Any suitable antibody can be used in the context of the invention. For example, the monoclonal antibody J5 is a murine IgG2a antibody that is specific for Common Acute Lymphoblastic Leukemia Antigen (CALLA) (Ritz et al., Nature, 283, 583-585 (1980)), and can be used to target cells that express CALLA (e.g., acute lymphoblastic leukemia cells). The monoclonal antibody MY9 is a murine IgG1 antibody that binds specifically to the CD33 antigen (Griffin et al., Leukemia Res., 8, 521 (1984)), and can be used to target cells that express CD33 (e.g., acute myelogenous leukemia (AML) cells).

Similarly, the monoclonal antibody anti-B4 (also referred to as B4) is a murine IgG1 antibody that binds to the CD19 antigen on B cells (Nadler et al., J. Immunol., 131, 244-250 (1983)), and can be used to target B cells or diseased cells that express CD19 (e.g., non-Hodgkin's lymphoma cells and chronic lymphoblastic leukemia cells). N901 is a murine monoclonal antibody that binds to the CD56 (neural cell adhesion molecule) antigen found on cells of neuroendocrine origin, including small cell lung tumor, which can be used in the conjugate to target drugs to cells of neuroendocrine origin. The J5, MY9, and B4 antibodies preferably are resurfaced or humanized prior to their use as part of the conjugate. Resurfacing or humanization of antibodies is described in, for example, Roguska et al., Proc. Natl. Acad. Sci. USA, 91, 969-73 (1994).

In addition, the monoclonal antibody C242 binds to the CanAg antigen (see, e.g., U.S. Patent 5,552,293), and can be used to target the conjugate to CanAg expressing tumors, such as colorectal, pancreatic, non-small cell lung, and gastric cancers. HuC242 is a humanized form of the monoclonal antibody C242 (see, e.g., U.S. Patent 5,552,293). The hybridoma from which it HuC242 is produced is deposited with ECACC identification Number 90012601. HuC242 can be prepared using CDR-grafting methodology (see, e.g., U.S. Patents 5,585,089, 5,693,761, and 5,693,762), or resurfacing technology (see, e.g., U.S. Patent 5,639,641). HuC242 can be used to target the conjugate to tumor cells expressing the CanAg antigen, such as, for example, colorectal, pancreatic, non-small cell lung, and gastric cancer cells.

To target ovarian cancer and prostate cancer cells, an anti-MUC1 antibody can be used as the cell binding agent in the conjugate. Anti-MUC1 antibodies include, for example, anti-HMFG-2 (see, e.g., Taylor-Papadimitriou et al., Int. J. Cancer, 28, 17-21 (1981)), hCTM01 (see, e.g., van Hof et al., Cancer Res., 56, 5179-5185 (1996)), or DS6. Prostate cancer cells also can be targeted with the conjugate by using an anti-prostate-specific membrane antigen (PSMA) as the cell binding agent, such as J591 (see, e.g., Liu et al., Cancer Res., 57, 3629-3634 (1997)). Moreover, cancer cells that express the Her2 antigen, such as breast, prostate, and ovarian cancers, can be targeted using the antibody trastuzumab. Anti-IGF-IR antibodies that bind to insulin-like growth factor receptor also can be used in conjugate.

Particularly preferred antibodies are humanized monoclonal antibodies, examples of which include huN901, huMy9-6, huB4, huC242, trastuzumab, bivatuzumab, sibrotuzumab, CNTO95, huDS6, and rituximab (see, e.g., U.S. Patents 5,639,641 and 5,665,357, U.S. Patent Application Publication 2005/0118183 A1, International (PCT) Patent Application PublicationWO 02/16401, Pedersen et al., *supra,* Roguska et al., *supra,* Liu et al., *supra,* Nadler et al., *supra,* Colomer et al., Cancer Invest., 19, 49-56 (2001), Heider et al., Eur. J. Cancer, 31A, 2385-2391 (1995), Welt et al., J. Clin. Oncol., 12, 1193-1203 (1994), and Maloney et al., Blood, 90, 2188-2195 (1997)). Most preferably, the antibody is the huN901 humanized monoclonal antibody or the huMy9-6 humanized monoclonal antibody. Other humanized monoclonal antibodies are known in the art and can be used in connection with the invention.

While the cell binding agent preferably is an antibody, the cell binding agent also can be a non-antibody molecule. Suitable non-antibody molecules include, for example, interferons (e.g., alpha, beta, or gamma interferon), lymphokines (e.g., interleukin 2 (IL-2), IL-3, IL-4, or IL-6), hormones (e.g., insulin), growth factors (e.g., EGF, TGF-alpha, FGF, and VEGF), colony-stimulating factors (e.g., G-CSF, M-CSF, and GM-CSF (see, e.g., Burgess, Immunology Today, 5, 155-158 (1984)), somatostatin, and transferrin (see, e.g., O'Keefe et al., J. Biol. Chem., 260, 932-937 (1985)). For example, GM-CSF, which binds to myeloid cells, can be used as a cell binding agent to target acute myelogenous leukemia cells. In addition, IL-2, which binds to activated T-cells, can be used for prevention of transplant graft rejection, for therapy and prevention of graft-versus-host disease, and for treatment of acute T-cell leukemia. Epidermal growth factor (EGF) can be used to target squamous cancers such as lung cancer and head and neck cancer. Somatostatin can be used to target neuroblastoma cells and other tumor cell types.

The conjugate can comprise any suitable drug, typically a cytotoxic agent. A "cytotoxic agent," as used herein, refers to any compound that results in the death of a cell, induces cell death, or decreases cell viability. Suitable cytotoxic agents include, for example, maytansinoids and maytansinoid analogs, taxoids, CC-1065 and CC-1065 analogs, and dolastatin and dolastatin analogs. In a preferred embodiment of the invention, the cytotoxic agent is a maytansinoid, including maytansinol and maytansinol analogs. Maytansinoids are compounds that inhibit microtubule formation and are highly toxic to mammalian cells. Examples of suitable maytansinol analogues include those having a modified aromatic ring and those having modifications at other positions. Such maytansinoids are described in, for example, U.S. Patents 4,256,746, 4,294,757, 4,307,016, 4,313,946, 4,315,929, 4,322,348, 4,331,598, 4,361,650, 4,362,663, 4,364,866, 4,424,219, 4,371,533, 4,450,254, 5,475,092, 5,585,499, 5,846,545, and 6,333,410.

Examples of maytansinol analogs having a modified aromatic ring include: (1) C-19-dechlora (U.S. Patent 4,256,746) (prepared by LAH reduction of ansamytocin P2), (2) C-20-hydroxy (or C-20-demethyl) +/-C-19-dechloro (U.S. Patents 4,361,650 and 4,307,016) (prepared by demethylation using *Streptomyces* or *Actinomyces* or dechlorination using LAH), and (3) C-20-demethoxy, C-20-acyloxy (-OCOR), +/-dechloro (U.S. Patent 4,294,757) (prepared by acylation using acyl chlorides).

Examples of maytansinol analogs having modifications of positions other than an aromatic ring include: (1) C-9-SH (U.S. Patent 4,424,219) (prepared by the reaction of maytansinol with H₂S or P₂S₅), (2) C-14-alkoxymethyl (demethoxy/CH₂OR) (U.S. Patent 4,331,598), (3) C-14-hydroxymethyl or acyloxymethyl (CH₂OH or CH₂OAc) (U.S. Patent 4,450,254) (prepared from *Nocardia*), (4) C-15-hydroxy/acyloxy (U.S. Patent 4,364,866) (prepared by the conversion of maytansinol by *Streptomyces*), (5) C-15-methoxy (U.S. Patents 4,313,946 and 4,315,929) (isolated from *Trewia nudiflora*), (6) C-18-N-demethyl (U.S. Patents 4,362,663 and 4,322,348) (prepared by the demethylation of maytansinol by *Streptomyces*), and (7) 4,5-deoxy (U.S. Patent 4,371,533) (prepared by the titanium trichloride/LAH reduction of maytansinol).

In a preferred embodiment of the invention, the conjugates utilize the thiol-containing maytansinoid DM1, also known as N2'-deacetyl-N2'-(3-mercapto-1-oxopropyl)-maytansine, as the cytotoxic agent. The structure of DM1 is represented by formula (I):

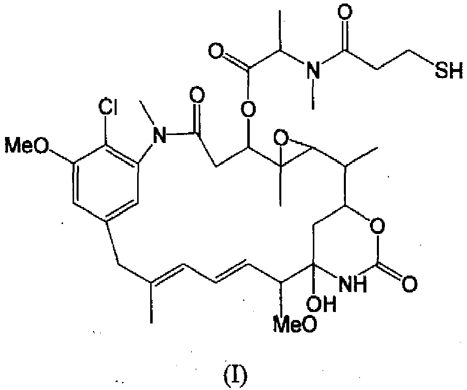

In another preferred embodiment of the invention, the conjugate utilizes the thiol-containing maytansinoid DM4, also known as N-2'-deacetyl-N-2'-(4-methyl-4-mercapto-1-oxopentyl)-maytansine, as the cytotoxic agent. The structure of DM4 is represented by formula (II):

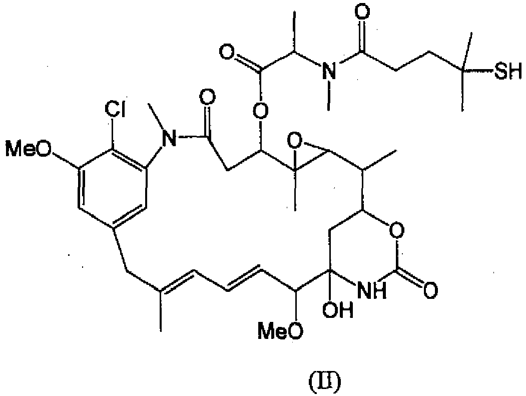

Other maytansines may be used in connection with the inventive process, including, for example, thiol and disulfide-containing maytansinoids bearing a mono or dialkyl substitution on the carbon atom bearing the sulfur atom. Particularly preferred is a maytansinoid having at the C₃ position (a) C₁₄ hydroxymethyl, C₁₅ hydroxy, or C₂₀ desmethyl functionality, and (b) an acylated amino acid side chain with an acyl group bearing a hindered sulfhydryl group, wherein the carbon atom of the acyl group bearing the thiol functionality has one or two substituents, said substituents being CH₃, C₂H₅, linear or branched alkyl or alkenyl having from 1 to 10 carbon atoms, cyclic alkyl or alkenyl having from 3 to 10 carbon atoms, phenyl, substituted phenyl, or heterocyclic aromatic or heterocycloalkyl radical, and further wherein one of the substituents can be H, and wherein the acyl group has a linear chain length of at least three carbon atoms between the carbonyl functionality and the sulfur atom.

Additional maytansines for use in the invention include compounds represented by formula (III): 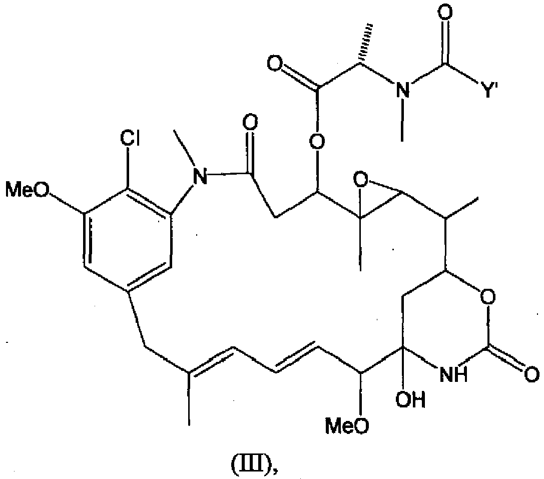 wherein Y' represents (CR₇CR₈)ₗ(CR₉=CR₁₀)ₚC=C_{q}Aᵣ(CR₅CR₆)ₘDᵤ(CR₁₁=CR₁₂)ᵣ(C=C)ₛBₜ(CR₃CR₄)ₙ-CR₁R₂SZ,
wherein R₁ and R₂ are each independently CH₃, C₂H₅, linear alkyl or alkenyl having from 1 to 10 carbon atoms, branched or cyclic alkyl or alkenyl having from 3 to 10 carbon atoms, phenyl, substituted phenyl or heterocyclic aromatic or heterocycloalkyl radical, and wherein R₂ also can be H,
wherein A, B, D are cycloalkyl or cycloalkenyl having 3-10 carbon atoms, simple or substituted aryl, or heterocyclic aromatic, or heterocycloalkyl radical,
wherein R₃, R₄, R₅, R₆, R₇, R₈, R₉, R₁₁, and R₁₂ are each independently H, CH₃, C₂H₅, linear alkyl or alkenyl having from 1 to 10 carbon atoms, branched or cyclic alkyl or alkenyl having from 3 to 10 carbon atoms, phenyl, substituted phenyl or heterocyclic aromatic, or heterocycloalkyl radical,
wherein l, m, n, o, p, q, r, s, and t are each independently zero or an integer from 1 to 5, provided that at least two of l, m, n, o, p, q, r, s and t are not zero at any one time, and
wherein Z is H, SR or COR, wherein R is linear alkyl or alkenyl having from 1 to 10 carbon atoms, branched or cyclic alkyl or alkenyl having from 3 to 10 carbon atoms, or simple or substituted aryl or heterocyclic aromatic, or heterocycloalkyl radical.

Preferred embodiments of formula (III) include compounds of formula (III) wherein (a) R₁ is H, R₂ is methyl and Z is H, (b) R₁ and R₂ are methyl and Z is H, (c) R₁ is H, R₂ is methyl, and Z is -SCH₃, and (d) R₁ and R₂ are methyl, and Z is -SCH₃.

Such additional maytansines also include compounds represented by formula (IV-L), (IV-D), or (IV-D,L): 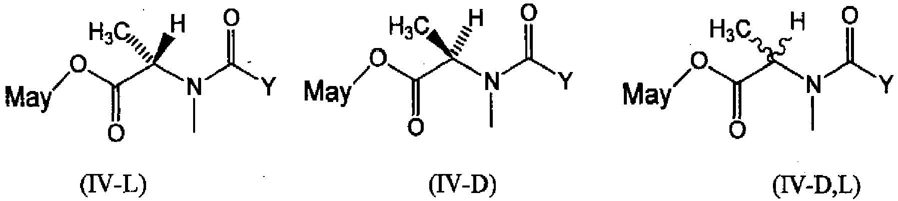 wherein Y represents (CR₇CR₈)ₗ(CR₅CR₆)ₘ(CR₃CR₄)ₙCR₁R₂SZ,
wherein R₁ and R₂ are each independently CH₃, C₂H₅, linear alkyl, or alkenyl having from 1 to 10 carbon atoms, branched or cyclic alkyl or alkenyl having from 3 to 10 carbon atoms, phenyl, substituted phenyl, or heterocyclic aromatic or heterocycloalkyl radical, and wherein R₂ also can be H,
wherein R₃, R₄, R₅, R₆, R₇, and R₈ are each independently H, CH₃, C₂H₅, linear alkyl or alkenyl having from 1 to 10 carbon atoms, branched or cyclic alkyl or alkenyl having from 3 to 10 carbon atoms, phenyl, substituted phenyl, or heterocyclic aromatic or heterocycloalkyl radical,
wherein 1, m, and n are each independently an integer of from 1 to 5, and in addition n can be zero,
wherein Z is H, SR, or COR wherein R is linear or branched alkyl or alkenyl having from 1 to 10 carbon atoms, cyclic alkyl or alkenyl having from 3 to 10 carbon atoms, or simple or substituted aryl or heterocyclic aromatic or heterocycloalkyl radical, and
wherein May represents a maytansinoid which bears the side chain at C-3, C-14 hydroxymethyl, C-15 hydroxy, or C-20 desmethyl.

Preferred embodiments of formulas (IV-L), (IV-D) and (IV-D,L) include compounds of formulas (IV-L), (IV-D) and (IV-D,L) wherein (a) R₁ is H, R₂ is methyl, R₅, R₆, R₇, and R₈ are each H, l and m are each 1, n is 0, and Z is H, (b) R₁ and R₂ are methyl, R₅, R₆, R₇, R₈ are each H, l and m are 1, n is 0, and Z is H, (c) R₁ is H, R₂ is methyl, R₅, R₆, R₇, and R₈ are each H, 1 and m are each 1, n is 0, and Z is -SCH₃, or (d) R₁ and R₂ are methyl, R₅, R₆, R₇, R₈ are each H, l and m are 1, n is 0, and Z is -SCH₃.

Preferably the cytotoxic agent is represented by formula (IV-L).

Additional preferred maytansines also include compounds represented by formula (V): 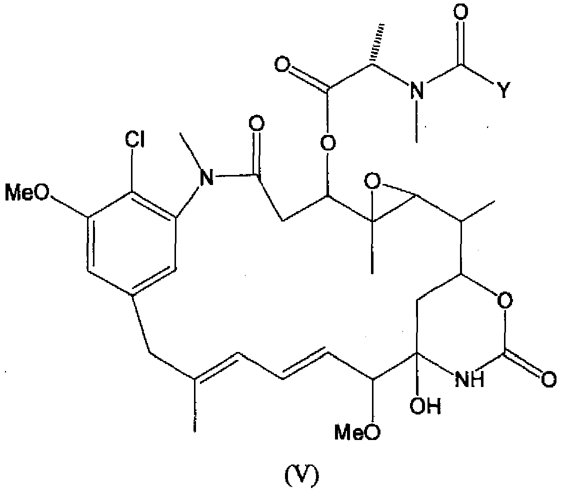 wherein Y represents (CR₇CR₈)ₗ(CR₅CR₆)ₘ(CR₃CR₄)ₙCR₁R₂SZ,
wherein R1 and R2 are each independently CH₃, C₂H₅, linear alkyl, or alkenyl having from 1 to 10 carbon atoms, branched or cyclic alkyl or alkenyl having from 3 to 10 carbon atoms, phenyl, substituted phenyl or heterocyclic aromatic or heterocycloalkyl radical, and wherein R₂ also can be H,
wherein R₃, R₄, R₅, R₆, R₇, and R₈ are each independently H, CH₃, C₂H₅, linear alkyl or alkenyl having from 1 to 10 carbon atoms, branched or cyclic alkyl or alkenyl having from 3 to 10 carbon atoms, phenyl, substituted phenyl, or heterocyclic aromatic or heterocycloalkyl radical,
wherein 1, m, and n are each independently an integer of from 1 to 5, and in addition n can be zero, and
wherein Z is H, SR or COR, wherein R is linear alkyl or alkenyl having from 1 to 10 carbon atoms, branched or cyclic alkyl or alkenyl having from 3 to 10 carbon atoms, or simple or substituted aryl or heterocyclic aromatic or heterocycloalkyl radical.

Preferred embodiments of formula (V) include compounds of formula (V) wherein (a) R₁ is H, R₂ is methyl, R₅, R₆, R₇, and R₈ are each H; l and m are each 1; n is 0; and Z is H, (b) R₁ and R₂ are methyl; R₅, R₆, R₇, R₈ are each H, l and m are 1; n is 0; and Z is H, (c) R₁ is H, R₂ is methyl, R₅, R₆, R₇, and R₈ are each H, l and m are each 1, n is 0, and Z is -SCH₃, or (d) R₁ and R₂ are methyl, R₃, R₆, R₇, R₈ are each H, l and m are 1, n is 0, and Z is -SCH₃.

Still further preferred maytansines include compounds represented by formula (VI-L), (VI-D), or (VI-D,L): 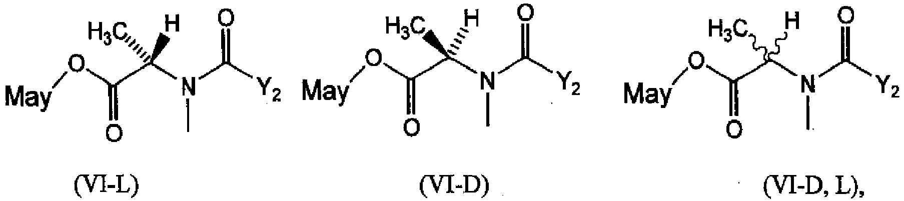 wherein Y₂ represents (CR₇CR₈)ₗ(CR₅CR₆)ₘ(CR₃CR₄₎nCR₁R₂SZ₂,
wherein R₁ and R₂ are each independently CH₃, C₂H₅, linear alkyl or alkenyl having from 1 to 10 carbon atoms, branched or cyclic alkyl or alkenyl having from 3 to 10 carbon atoms, phenyl, substituted phenyl or heterocyclic aromatic or heterocycloalkyl radical, and wherein R₂ also can be H,
wherein R₃, R₄, R₅, R₆, R₇, and R₈ are each independently H, CH₃, C₂H₅, linear cyclic alkyl or alkenyl having from 1 to 10 carbon atoms, branched or cyclic alkyl or alkenyl having from 3 to 10 carbon atoms, phenyl, substituted phenyl or heterocyclic aromatic or heterocycloalkyl radical,
wherein l, m, and n are each independently an integer of from 1 to 5, and in addition n can be zero,
wherein Z₂ is SR or COR, wherein R is linear alkyl or alkenyl having from 1 to 10 carbon atoms, branched or cyclic alkyl or alkenyl having from 3 to 10 carbon atoms, or simple or substituted aryl or heterocyclic aromatic or heterocycloalkyl radical, and
wherein May is a maytansinoid.

Additional preferred maytansines include compounds represented by formula (VII): 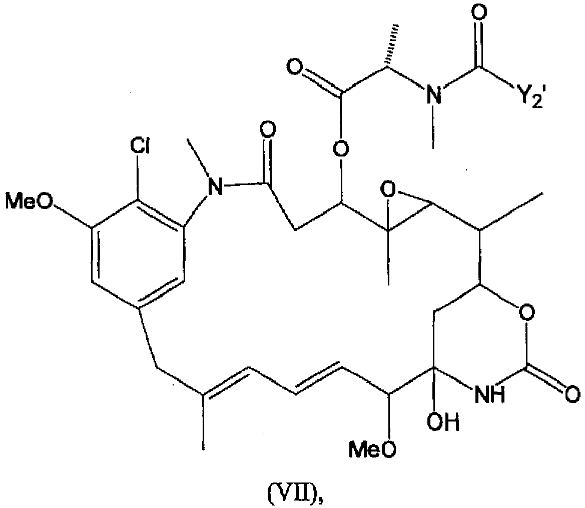 wherein Y₂, represents (CR₇CR₈)ₗ(CR₉=CR₁₀)ₚ(C=C)_{q}Ar(CR₅CR₆)ₘDu(CR₁₁=CR₁₂)ᵣ(C=C)sBt(CR₃CR₄)ₙCR₁R₂SZ2,
wherein R₁ and R₂ are each independently CH₃, C₂H₅, linear branched or alkyl or alkenyl having from 1 to 10 carbon atoms, cyclic alkyl or alkenyl having from 3 to 10 carbon atoms, phenyl, substituted phenyl or heterocyclic aromatic or heterocycloalkyl radical, and in addition R₂ can be H,
wherein A, B, and D each independently is cycloalkyl or cycloalkenyl having 3 to 10 carbon atoms, simple or substituted aryl, or heterocyclic aromatic or heterocycloalkyl radical,
wherein R₃, R₄, R₅, R₆, R₇, R₈, R₉, R₁₁, and R₁₂ are each independently H, CH₃, C₂H₅, linear alkyl or alkenyl having from 1 to 10 carbon atoms, branched or cyclic alkyl or alkenyl having from 3 to 10 carbon atoms, phenyl, substituted phenyl or heterocyclic aromatic or heterocycloalkyl radical,
wherein l, m, n, o, p, q, r, s, and t are each independently zero or an integer of from 1 to 5, provided that at least two of l, m, n, o, p, q, r, s and t are not zero at any one time, and
wherein Z₂ is SR or -COR, wherein R is linear alkyl or alkenyl having from 1 to 10 carbon atoms, branched or cyclic alkyl or alkenyl having from 3 to 10 carbon atoms, or simple or substituted aryl or heterocyclic aromatic or heterocycloalkyl radical.

Preferred embodiments of formula (VII) include compounds of formula (VII), wherein R₁ is H and R₂ is methyl.

In addition to maytansinoids, the cytotoxic agent used in the conjugate can be a taxane or derivative thereof. Taxanes are a family of compounds that includes paclitaxel (TAXOL®), a cytotoxic natural product, and docetaxel (TAXOTERE™), a semi-synthetic derivative, which are both widely used in the treatment of cancer. Taxanes are mitotic spindle poisons that inhibit the depolymerization of tubulin, resulting in cell death. While docetaxel and paclitaxel are useful agents in the treatment of cancer, their antitumor activity is limited because of their non-specific toxicity towards normal cells. Further, compounds like paclitaxel and docetaxel themselves are not sufficiently potent to be used in conjugates of cell binding agents.

A preferred taxane for use in the preparation of cytotoxic conjugates is the taxane of formula (VIII):

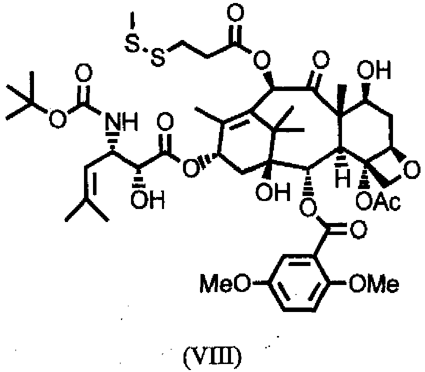

Methods for synthesizing taxanes that can be used in the conjugates of the present invention, along with methods for conjugating taxanes to cell binding agents such as antibodies, are described in detail in U.S. Patents 5,416,064, 5,475,092, 6,340,701, 6,372,738, 6,436,931, 6,596,757, 6,706,708, and 6,716,821, and in U.S. Patent Application Publication No. 2004/0024049 A1.

The cytotoxic also can be CC-1065 or a derivative thereof. CC-1065 is a potent anti-tumor antibiotic isolated from the culture broth of *Streptomyces zelensis*. CC-1065 is about 1000-fold more potent *in vitro* than commonly used anti-cancer drugs, such as doxorubicin, methotrexate, and vincristine (Bhuyan et al., Cancer Res., 42, 3532-3537 (1982)). CC-1065 and its analogs are disclosed in U.S. Patents 5,585,499, 5,846,545, 6,340,701, and 6,372,738. The cytotoxic potency of CC-1065 has been correlated with its alkylating activity and its DNA-binding or DNA-intercalating activity. These two activities reside in separate parts of the molecule. In this respect, the alkylating activity is contained in the cyclopropapyrroloindole (CPI) subunit and the DNA-binding activity resides in the two pyrroloindole subunits of CC-1065.

Several CC-1065 analogs are known in the art and also can be used as the cytotoxic agent in the conjugate (see, e.g., Warpehoski et al., J. Med. Chem., 31, 590-603 (1988)). A series of CC-1065 analogs has been developed in which the CPI moiety is replaced by a cyclopropabenzindole (CBI) moiety (Boger et al., J. Org. Chem., 55, 5823-5833 (1990), and Boger et al., Bioorg. Med. Chem. Lett., 1, 115-120 (1991)). These CC-1065 analogs maintain the high *in vitro* potency of the parental drug, without causing delayed toxicity in mice. Like CC-1065, these compounds are alkylating agents that covalently bind to the minor groove of DNA to cause cell death.

The therapeutic efficacy of CC-1065 analogs can be greatly improved by changing the *in vivo* distribution through targeted delivery to a tumor site, resulting in lower toxicity to non-targeted tissues, and thus, lower systemic toxicity. To this end, conjugates of analogs and derivatives of CC-1065 with cell binding agents that specifically target tumor cells have been generated (see, e.g., U.S. Patents 5,475,092, 5,585,499, and 5,846,545). These conjugates typically display high target-specific cytotoxicity *in vitro,* and anti-tumor activity in human tumor xenograft models in mice (see, e.g., Chari et al., Cancer Res., 55, 4079-4084 (1995)).

Methods for synthesizing CC-1065 analogs are described in detail in U.S. Patents 5,475,092, 5,585,499, 5,846,545, 6,534,660, 6,586,618, and 6,756,397 and U.S. Patent Application Publication 2003/0195365 A1.

Drugs such as methotrexate, daunorubicin, doxorubicin, vincristine, vinblastine, melphalan, mitomycin C, chlorambucil, calicheamicin, tubulysin and tubulysin analogs, duocarmycin and duocarmycin analogs, dolastatin and dolastatin analogs also can be used in accordance with the inventive process. Doxarubicin and daunorubicin compounds (see, e.g., U.S. Patent 6,530,579) can also be used as the drug.

The drug conjugates may be prepared by *in vitro* methods. In order to link a drug or prodrug to the antibody, a linking group is used. Suitable linking groups are well known in the art and include disulfide groups, acid labile groups, photolabile groups, peptidase labile groups, and esterase labile groups. Preferred linking groups are disulfide groups. For example, conjugates can be constructed using a disulfide exchange reaction between the antibody and the drug or prodrug. The drug molecules also can be linked to a cell binding agent through an intermediary carrier molecule such as serum albumin.

In accordance with the inventive method, the cell binding agent is modified by reacting a bifunctional crosslinking reagent with the cell binding agent, thereby resulting in the covalent attachment of a linker molecule to the cell binding agent. As used herein, a "bifunctional crosslinking reagent" is any chemical moiety that covalently links a cell binding agent to a drug, such as the drugs described herein. In a preferred embodiment of the invention, a portion of the linking moiety is provided by the drug. In this respect, the drug comprises a linking moiety that is part of a larger linker molecule that is used to join the cell binding agent to the drug. For example, to form the maytansinoid DM1, the side chain at the C-3 hydroxyl group of maytansine is modified to have a free sulfhydryl group (SH). This thiolated form of maytansine can react with a modified cell-binding agent to form a conjugate. Therefore, the final linker is assembled from two components, one of which is provided by the crosslinking reagent, while the other is provided by the side chain from DM1.

Any suitable bifunctional crosslinking reagent can be used in connection with the invention, so long as the linker reagent provides for retention of the therapeutic, e.g., cytotoxicity, and targeting characteristics of the drug and the cell binding agent, respectively. Preferably, the linker molecule joins the drug to the cell binding agent through chemical bonds (as described above), such that the drug and the cell binding agent are chemically coupled (e.g., covalently bonded) to each other. Preferably, the linking reagent is a cleavable linker. More preferably, the linker is cleaved under mild conditions, i.e., conditions within a cell under which the activity of the drug is not affected. Examples of suitable cleavable linkers include disulfide linkers, acid labile linkers, photolabile linkers, peptidase labile linkers, and esterase labile linkers. Disulfide containing linkers are linkers cleavable through disulfide exchange, which can occur under physiological conditions. Acid labile linkers are linkers cleavable at acid pH. For example, certain intracellular compartments, such as endosomes and lysosomes, have an acidic pH (pH 4-5), and provide conditions suitable to cleave acid labile linkers. Photo labile linkers are useful at the body surface and in many body cavities that are accessible to light. Furthermore, infrared light can penetrate tissue. Peptidase labile linkers can be used to cleave certain peptides inside or outside cells (see e.g., Trouet et al., Proc. Natl. Acad Sci. USA, 79, 626-629 (1982), and Umemoto et al., Int. J. Cancer, 43, 677-684 (1989)).

Preferably the drug is linked to a cell binding agent through a disulfide bond. The linker molecule comprises a reactive chemical group that can react with the cell binding agent. Preferred reactive chemical groups for reaction with the cell binding agent are *N-*succinimidyl esters and *N*-sulfosuccinimidyl esters. Additionally the linker molecule comprises a reactive chemical group, preferably a dithiopyridyl group, that can react with the drug to form a disulfide bond. Particularly preferred linker molecules include, for example, N-succinimidyl 3-(2-pyridyldithio)propionate (SPDP) (see, e.g., Carlsson et al., Biochem. J., 173, 723-737 (1978)), N-succinimidyl 4-(2-pyridyldithio)butanoate (SPDB) (see, e.g., U.S. Patent 4,563,304) and N-succinimidyl 4-(2-pyridyldithio)pentanoate (SPP) (see, e.g., CAS Registry number 341498-08-6).

While cleavable linkers preferably are used in the inventive process, a non-cleavable linker also can be used to generate the above-described conjugate. A non-cleavable linker is any chemical moiety that is capable of linking a drug, such as a maytansinoid, a taxane, or a CC-1065 analog, to a cell binding agent in a stable, covalent manner. Thus, non-cleavable linkers are substantially resistant to acid-induced cleavage, light-induced cleavage, peptidase-induced cleavage, esterase-induced cleavage, and disulfide bond cleavage, at conditions under which the drug or the cell binding agent remains active.

Suitable crosslinking reagents that form non-cleavable linkers between a drug and the cell-binding agent are well known in the art. Examples of non-cleavable linkers include linkers having an *N*-succinimidyl ester or *N*-sulfosuccinimidyl ester moiety for reaction with the cell binding agent, as well as a maleimido- or haloacetyl-based moiety for reaction with the drug. Crosslinking reagents comprising a maleimido-based moiety include N-succinimidyl 4-(maleimidomethyl)cyclohexanecarboxylate (SMCC), N-succinimidyl-4-(N-maleimidomethyl)-cyclohexane-1-carboxy-(6-amidocaproate), which is a "long chain" analog of SMCC (LC-SMCC), κ-maleimidoundecanoic acid N-succinimidyl ester (KMUA), γ-maleimidobutyric acid N-succinimidyl ester (GMBS), ε-maleimidocaproic acid N-hydroxysuccinimide ester (EMCS), m-maleimidobenzoyl-N-hydroxysuccinimide ester (MBS), N-(α-maleimidoacetoxy)-succinimide ester (AMAS), succinimidyl-6-(β-maleimidopropionamido)hexanoate (SMPH), N-succinimidyl 4-(p-maleimidophenyl)-butyrate (SMPB), and N-(p-maleimidophenyl)isocyanate (PMPI). Cross-linking reagents comprising a haloacetyl-based moiety include N-succinimidyl-4-(iodoacetyl)-aminobenzoate (SIAB), N-succinimidyl iodoacetate (SIA), N-succinimidyl bromoacetate (SBA), and N-succinimidyl 3-(bromoacetamido)propionate (SBAP).

Other crosslinking reagents lacking a sulfur atom that form non-cleavable linkers can also be used in the inventive method. Such linkers can be derived from dicarboxylic acid based moieties. Suitable dicarboxylic acid based moieties include, but are not limited to, α,ω-dicarboxylic acids of the general formula (IX):

HOOC-Xₗ-Yₙ-Zₘ-COOH (IX),

wherein X is a linear or branched alkyl, alkenyl, or alkynyl group having 2 to 20 carbon atoms, Y is a cycloalkyl or cycloalkenyl group bearing 3 to 10 carbon atoms, Z is a substituted or unsubstituted aromatic group bearing 6 to 10 carbon atoms, or a substituted or unsubstituted heterocyclic group wherein the hetero atom is selected from N, O or S, and wherein l, m, and n are each 0 or 1, provided that 1, m, and n are all not zero at the same time.

Many of the non-cleavable linkers disclosed herein are described in detail in U.S. Patent Application Publication 2005/0169933 A1.

In accordance with the inventive process, a mixture is produced comprising the cell binding agent having linkers stably and unstably bound thereto, as well as reactants and other by-products. A linker is "stably" bound to the cell binding agent when the covalent bond between the linker and the cell binding agent is not substantially weakened or severed under normal storage conditions over a period of time, which could range from a few months to a few years. In contrast, a linker is "unstably" bound to the cell binding agent when the covalent bond between the linker and the cell binding agent is substantially weakened or severed under normal storage conditions over a period of time, which could range from few months to few years. Purification of the modified cell binding agent from reactants and by-products preferably is carried out by subjecting the mixture to SEPHADEX™ resin chromatography or a similar non-absorptive chromatographic step. Examples of suitable chromatography resins include, but are not limited to, SEPHADEX™ G-25, G-50, G-100, SEPHACRYL™ resins (e.g., S-200 and S-300), SUPERDEX™ resins (e.g., SUPERDEX™ 75 and SUPERDEX™ 200), BIO-GEL® resins (e.g., P-6, P-10, P-30, P-60, and P-100), and others known to those of ordinary skill in the art.

The modified cell binding agent is conjugated to a drug (e.g., a maytansinoid) by reacting the modified cell binding agent with the drug in a solution having pH ranging from pH about 4.5 to about 8, wherein the conjugation step results in formation of a mixture of stable cell binding agent-drug conjugates, non-stable cell binding agent-drug conjugates, non-conjugated drug (i.e., "free" drug), reactants, and by-products. Further purification can be carried out by repeating the purification step, e.g., on SEPHADEX™ G-25 or a similar non-absorptive chromatographic resin, described above, to remove the non-conjugated drug, reactants, and by-products and to substantially retain the cell binding agent-drug conjugates.

The inventive process further comprises a holding step after modification of the cell binding agent with a bifunctional crosslinking reagent. The holding step comprises maintaining the solution at a suitable temperature for a suitable period of time to release the unstably bound linkers from the cell binding agent while not substantially releasing the stably bound linkers from the cell binding agent. Desirably, the holding step comprises maintaining the solution at a temperature of about 2 °C to about 8 °C for a period of at least about 12 hours for up to 30 days or more. Alternatively, the duration of the holding step can be substantially reduced by performing the holding step at elevated temperature, with the maximum temperature limited by the stability of the cell binding agent-drug conjugate. For example, for an antibody-drug conjugate the holding step can be performed at up to about 37 °C for up to about four weeks, preferably between two to four weeks, even more preferably, between one and two weeks, and most preferably for about one week or less (e.g., 2 hours to about six days). Preferred pH values for the holding step range from about 6-10. Most preferred pH values are between about 6.5 and 8.5. Preferably, the holding step comprises incubating the mixture comprising the modified cell binding agent at 4 °C at pH 6.5 for at least about 12 hours to 4 weeks. More preferably, the holding step comprises incubating the mixture comprising the modified cell binding agent at a range between 20-30 °C at pH 6.5 for about 12 hours to about 1 week. The holding step can be performed before or after the cell binding agent is conjugated to the drug. Preferably, the holding step is performed directly after the modification of the cell binding agent with the bifunctional crosslinking reagent.

In a preferred embodiment the inventive process comprises a holding step after modification of the cell binding agent with a bifunctional crosslinking reagent and before conjugation. The holding step comprises maintaining the solution at a suitable temperature for a suitable period of time to release the unstably bound linkers from the cell binding agent while not substantially releasing the stably bound linkers from the cell binding agent. Desirably, the holding step comprises maintaining the solution at a temperature of about 2° C to about 8° C for a period of at least about 5 hours for up to 5 days or more, for example 30 days. Alternatively, the duration of the holding step can be substantially reduced by performing the holding step at elevated temperature, with the maximum temperature limited by the stability of the cell binding agent-drug conjugate. For example, for an antibody-drug conjugate the holding step can be performed at up to about 37° C for up to about four weeks, preferably between two to four weeks, even more preferably, between one and two weeks, and most preferably for about one week or less (e.g., 2 hours to about six days). The pH value for the holding step preferably is about 4 or more, but less than about 6 (e.g., 4-5.9). The pH value for the holding step more preferably is about 5 or more, but less than about 6 (e.g., 5-5.9). Preferably, the holding step comprises incubating the mixture comprising the modified cell binding agent at 4° C at pH 5 for at least about 5 hours for up to 5 days or more, for example 10 days. More preferably, the holding step comprises incubating the mixture comprising the modified cell binding agent at a range between 20-30° C at a pH of about 5 for about 5 hours to about 1 to 3 days, preferably 1 day. After modification of the cell binding agent, a purification step may be performed before the hold step and/or after the hold step, but prior to the conjugation step. Such purification steps, such as non-adsorptive or adsorptive chromatography, are well known to one of ordinary skill in the art.

In one embodiment, the duration of the holding step can be substantially reduced by the addition of nucleophiles. The nucleophiles can be added during the conjugation step and the holding step can performed simultaneously with conjugation. In the context of the invention, nucleophiles are chemical moieties that can react with ester groups and imidazole amides on modified cell binding agents in aqueous solutions. Suitable nucleophiles are known in the art and include, for example, primary amines, i.e., RNH₂, where R is an alkyl or aromatic group; or secondary amines, i.e., RR'NH, where R and R' are alkyl or aromatic groups. Amines also can be amino acids, peptides containing lysine amino acids, peptides containing alpha-amino or non-natural secondary amino groups, or water-soluble amines. Nucleophiles can be in solution, in a polymeric state, or in an immobilized form as a solid phase reagent. Examples of suitable nucleophiles in solution include, but are not limited to, glycylglycine, glycine, taurine (sodium 2-aminoethanesulfonate), ethanolamine, diethanolamine, lysine, hydroxylamine, hydrazine, imidazole, histidine, ethylamine, 2-amino-2-(hydroxymethyl)-1,3-propane-diol and 4-amino-1-butanol. Appropriate concentrations of soluble nucleophiles range from about 0.1 mM to the limit of solubility for the particular nucleophile. Examples of nucleophiles in a polymeric state include, but are not limited to, poly(ethyleneimine), poly-lysine, and peptides with lysine amino acids and peptides containing alpha-amino or non-natural secondary amino groups. Examples of nucleophiles in immobilized form as a solid phase reagent include, but are not limited to, solid phase linked amines, such as EAH Sepharose 4B and aminomethyl polystyrene beads: For a solid phase nucleophile, the molar amount of solid phase amine should be in excess over the amount of crosslinker bound to the cell binding agent.

The inventive method further comprises conjugating the modified cell binding agent to a drug by reacting the modified cell binding agent with a drug in a solution comprising a pH from about 4.5 to about 8, whereupon a second mixture comprising (i) the cell binding agent chemically coupled to the drug, (ii) free drug, and (iii) solvents and reaction by products is produced. While the conjugation reaction is performed at a pH between about pH 4.5 to about pH 8.0, the reaction preferably is performed at a pH below 6 or greater than 7.

The inventive process may optionally include the addition of sucrose to the conjugation step used in the inventive process to increase solubility and recovery of the cell binding agent-drug conjugates. Desirably, sucrose is added at a concentration of about 0.1% (w/v) to about 20% (w/v) (e.g., about 0.1% (w/v), 1% (w/v), 5% (w/v), 10% (w/v), 15% (w/v), or 20% (w/v)). Preferably, sucrose is added at a concentration of about 1% (w/v) to 10% (w/v) (e.g., about 2% (w/v), about 4% (w/v), about 6% (w/v), or about 8% (w/v)). In addition, the conjugation reaction also can comprise the addition of a buffering agent. Any suitable buffering agent known in the art can be used. Suitable buffering agents include, for example, a citrate buffer, an acetate buffer, a succinate buffer, and a phosphate buffer.

Following the conjugation step, the conjugate preferably is subjected to a final purification step. In this regard, the conjugation mixture can be purified using a membrane-based tangential flow filtration process (TFF). One of ordinary skill in the art will appreciate that the final purification step enables the isolation of a stable conjugate comprising the cell binding agent chemically coupled to the drug.

Alternatively, as disclosed in U.S. Patent 6,441,163 B1, the drug can be first modified to introduce a reactive ester suitable to react with a cell-binding agent. Reaction of these maytansinoids containing an activated linker moiety with a cell-binding agent provides another method of producing a cleavable or non-cleavable cell-binding agent maytansinoid conjugate.

Thus, the invention also provides a process for preparing a conjugate comprising a cell binding agent chemically coupled to a drug, which process comprises: (a) contacting a cell binding agent with a drug bearing an active ester and thereby prepare a mixture comprising cell binding agents having drugs stably and unstably bound thereto, (b) subjecting the mixture to non-adsorptive chromatography to purify the cell binding agent having drug bound thereto from other components of the mixture and thereby prepare a purified mixture, (c) optionally subjecting the purified mixture to tangential flow filtration (TFF) to isolate a conjugate comprising the cell binding agent chemically coupled to the drug, and (d) holding the mixture between at least one of steps a-b and b-c to release the unstably bound drugs from the cell binding agent.

The conjugates can be purified as described in U.S. Patent 6,441,163 B1. After the conjugate is formed and before a final purification step, a holding step may be included in order to allow the isolation of a stable conjugate comprising the cell binding agent chemically coupled to the drug. The holding step may be reduced by performing at elevated temperature, at elevated pH, and/or in the presence of nucleophiles, as described above. In addition, reaction of the cell binding agent with the drug bearing a reactive ester may be performed at a pH of about 4.5 to about 8.0, optionally in the presence of sucrose (0.1 % (w/v) to about 20% (w/v)), also as described above.

The following examples further illustrate the invention but, of course, should not be construed as in any way limiting its scope.

### EXAMPLE 1A

This example demonstrates a process of preparing a conjugate comprising an antibody chemically coupled to a drug, which includes a holding step performed after modification of the antibody with a bifunctional crosslinking reagent and before conjugation of the antibody to the drug.

The huN901 monoclonal antibody (final concentration 8 mg/mL) was incubated with N-succinimidyl 4-(2-pyridyldithio)pentanoate (SPP) (7 fold molar excess of SPP) for approximately 100 minutes at room temperature in 50 mM potassium phosphate buffer (pH 6.5) containing 50 mM NaCl, 2 mM EDTA, and 5% ethanol. The reaction mixture was purified using a column of SEPHADEX™ G25F equilibrated and eluted in the aforementioned potassium phosphate buffer lacking ethanol.

Samples of modified antibody were held for up to two weeks at 4 ° C, then conjugated with the maytansinoid DM1 (1.7 fold molar excess over linker) dissolved in dimethylacetamide (DMA, final concentration is 3%). After overnight incubation at room temperature, the conjugated antibody samples were purified by chromatography on SEPHADEX™ G25F equilibrated in phosphate buffered saline (PBS), pH 6.5. Samples were stored at 4 °C for one week. The number of DM1 molecules linked per antibody molecule was determined using the previously reported extinction coefficients for antibody and drug (Liu et al., Proc. Natl. Acad. Sci. USA, 93, 8618-8623 (1996)). The amount of free drug present after the conjugation reaction was determined by injecting 20 - 50 µg conjugate onto a HiSep column equilibrated in 25% acetonitrile in 100 mM ammonium acetate buffer, pH 7.0, and eluting in acetonitrile. The peak area of total free drug species (eluted in the gradient and identified by comparison of elution time with known standards), which was measured using an absorbance detector set to a wavelength of 252 nm, was compared with the peak area related to bound drug (eluted in the conjugate peak in the column flow-through fractions) to calculate the proportion of total drug species that was free. The results of this analysis are shown in Table 1.

**Table 1: Effect of Hold Time on Conjugate Characteristics**

| **Hold Time** | **Drug/Antibody Ratio** | **% Free Drug** |
|---|---|---|
| 0 | 3.7 | 6.4 |
| 2 weeks | 3.6 | 0.5 |

As shown by the data set forth in Table 1, holding the modified antibody at 4 ° C, after modification but prior to conjugation, significantly reduced the amount of free drug in the conjugate sample after storage for one week, without significantly affecting the amount of drug that was bound (as reflected in the drug/antibody ratio).

### EXAMPLE 1B

This example demonstrates the beneficial impact of a holding step at pH 5.0 relative to pH 6.5 and 7.5 after modification of an antibody with a bifunctional crosslinking reagent and before conjugation of the modified antibody to the drug.

The huN901 monoclonal antibody (final concentration 8 mg/mL) was incubated with N-succinimidyl 4-(2-pyridyldithio)pentanoate (SPP) (5.6 fold molar excess of SPP) for approximately 180 minutes at 20 °C in 50 mM potassium phosphate buffer containing 50 mM NaCl, 2 mM EDTA, and 5% ethanol, at pH 7.5. The reaction mixture was split into three parts, and each part was purified using gel filtration columns packed with SEPHADEX™ G25 (NAP 10 column obtained from Amersham Biosciences) and equilibrated and eluted with three different buffers. The first column was equilibrated and eluted with a 50 mM potassium phosphate buffer (pH 7.5) containing 50 mM NaCl, and 2 mM EDTA. The second column was equilibrated and eluted with a 50 mM potassium phosphate buffer (pH 6.5) containing 50 mM NaCl and 2 mM EDTA. The third column was equilibrated and eluted with a 50 mM sodium citrate buffer (pH 5.0) containing 50 mM NaCl and 50 mM EDTA.

The number of pyridyldithio groups (-SSPy) linked to the antibody was assayed by treatment with dithiothreitol to release pyridine-2-thione, which has an extinction coefficient of 8,080 M⁻¹cm⁻¹ at 343 nM. All three samples had 4.7 -SSPy linked per molecule of antibody.

Samples of modified antibody were held for up to 121 hours at room temperature. The amount of 4-(2-pyridyldithio)pentanoic acid (PPA) released during the course of this time is a measure of release of weakly bound linker. PPA release was determined by injecting 45-55 µg of the modified antibodies every 1.5 hours onto a HiSep column equilibrated in 25% acetonitrile in 100 mM ammonium acetate buffer, pH 7.0, and eluting in acetonitrile. The peak area of the PPA species (eluted in the gradient and identified by comparison of elution time with known standards), which was measured using an absorbance detector set to a wavelength of 252 nm, was used to calculate the percentage of released linker in each sample. The results of this analysis are shown in the Figure.

As shown in the Figure, the release of PPA, which represents weakly bound linker, is significantly more rapid at pH 5.0, compared to pH 6.5 and 7.5. Furthermore, the release of PPA is substantially more complete at pH 5.0 over a period of time that is appropriate for manufacturing (e.g., between about 5 and 24 hours).

### EXAMPLE 2

This example demonstrates a process of preparing a conjugate comprising an antibody chemically coupled to a drug, which includes a holding step performed after modification of the antibody with a bifunctional crosslinking reagent and before conjugation of the antibody to the drug.

A solution of huC242 antibody (16 mg at 8 mg/mL) in a buffer at pH 6.5, consisting of 50 mM potassium phosphate, 50 mM sodium chloride, and 2 mM ethylenediaminetetraacetic acid (EDTA) disodium salt, was treated at ambient temperature with a solution of SPP (10 mM stock in ethanol, 6.5 molar excess, final ethanol concentration was 5% v/v). The reaction mixture was incubated at ambient temperature for 90 minutes. A portion (0.4 mL) of the mixture was then passed through a gel filtration column packed with SEPHADEX™ G25 (NAP 10 column obtained from Amersham Biosciences) to remove any unreacted SPP and other low molecular weight material. Elution with the above buffer gave the modified antibody ("sample A"). A second portion of the reaction mixture (0.2 mL) was treated with a solution of lysine (0.05 mL of a stock solution of 250 mM lysine in 500 mM potassium phosphate buffer pH 7.5, containing 10 mM EDTA) to give a final lysine concentration of 50 mM. The reaction mixture was incubated at ambient temperature for two hours and then purified by passage through a gel filtration column as described above to give "sample B".

The number of pyridyldithio groups (-SSPy) linked to the antibody was assayed by treatment with dithiothreitol to release pyridine-2-thione, which has an extinction coefficient of 8,080 M⁻¹cm⁻¹ at 343 nM. The parent sample A that had not been treated with lysine had 4.9 -SSPy linked per molecule of antibody. Sample B had 3.5 -SSPy linked per antibody molecule, indicating that lysine treatment removes unstably-bound linker, which in this sample was as much as 40% of the total initially bound linker.

The modified antibody samples A and B (2.3 mgs of antibody) were each diluted to a final concentration of 2.5 mg/mL in buffer at pH 6.5, consisting of 50 mM potassium phosphate, 50 mM sodium chloride, and 2 mM EDTA. Both samples were treated with DM1 in dimethylacaetamide (final concentration of dimethylacetamide 3% v/v) using a 1.7-fold molar excess of DM1 over -SSPy that were linked. Thus, sample A was treated with 0.13 micromole of DM1, while the lysine-treated sample B required only 0.086 micromole of DM1. The reaction mixtures were incubated for 24 h at ambient temperature.

The monomeric antibody-DM1 conjugates were separated by passage through gel filtration columns (20 mL) packed with SEPHACRYL™ S300, eluting with phosphate-buffered saline (PBS) at pH 6.5. The number of DM1 molecules linked per antibody molecule was determined as described in Example 1. Sample A, which originally contained 4.9 -SSPy groups, gave 3.5 DM1 per antibody, while sample B, which had 3.5 -SSPy resulted in 3.2 DM1 per antibody. Both antibody-DM1 conjugates were dialyzed into PBS for 48 hours, and re-assayed for DM1 content. In sample A, the number of DM1 molecules linked was reduced from 3.5 to 3.4, while the number of DM1 molecules linked in sample B remained unchanged at 3.2. This confirms the conclusion that treatments with lysine removes unstably bound linkers while having no effect on the level of stably bound DM1 drug that may be achieved.

### EXAMPLE 3

This example demonstrates a process of preparing a conjugate comprising an antibody chemically coupled to a drug, which comprises a holding step after purification of the conjugate reaction mixture and diafiltration (using membrane-based TFF) of the conjugate after the holding step.

The BIWA 4 antibody was modified with SPP (6.6-fold molar excess of SPP over antibody) for 105 minutes at room temperature in 50 mM potassium phosphate buffer, pH 6.5, containing 50 mM NaCl, 2 mM EDTA, and 5% ethanol. Modified antibody was then purified by chromatography on SEPHADEX™ G25F equilibrated in 50 mM potassium phosphate buffer, pH 6.5, containing 50 mM NaCl and 2 mM EDTA. The purified modified antibody was then conjugated with DM1 (1.7 fold molar excess over linker) in dimethylacetamide (DMA) (final concentration 3%). After incubation overnight at room temperature, the conjugate was purified by chromatography on SEPHADEX™ G25F equilibrated in PBS, pH 6.5.

One portion of the conjugate (Sample A) was placed at 4 ° C without further treatment. A second portion (Sample B) was held for four days at 4 ° C, then diafiltered against PBS, pH 6.5, and also placed at 4 °C. Free drug was measured at intervals as described in Example 1. The results are set forth in Table 2.

**Table 2: Effect of Holding on Released Free Drug**

| **Months at 4° C** | **Free Drug (%)** | | | | | | | |
|---|---|---|---|---|---|---|---|---|
| | **Sample A - No Diafiltration** | | | | **Sample B - 4 Day Hold, then Diafiltration** | | | |
| | **DM1** | **DM1-TPA** | **DM1 Dimer** | **Total Free Drug** | **DM1** | **DM1-TPA** | **DM1 Dimer** | **Total Free Drug** |
| 0 | 1.5 | 1.1 | 0.5 | 3.1 | 0.5 | 0.2 | 0.3 | 0.9 |
| 1 | 1.3 | 1.7 | 3.1 | 6.0 | 0.2 | 0.7 | 0.4 | 1.3 |
| 2 | 1.7 | 3.0 | 6.5 | 11.2 | 0.3 | 1.3 | 0.9 | 2.5 |
| 3 | 1.1 | 3.2 | 8.2 | 12.5 | 0.1 | 1.8 | 0.9 | 2.8 |

As shown by the data set forth in Table 2, release of free drug from the diafiltered sample was significantly reduced relative to release from the untreated sample.

### EXAMPLE 4A

This example demonstrates a process for preparing a conjugate comprising an antibody chemically coupled to a drug, which process comprises a prolonged hold step prior to diafiltration by membrane-based TFF.

The BIWA 4 monoclonal antibody (final concentration of 20 mg/mL) was reacted with SPP (6.6 fold molar excess of SPP) for approximately 130 minutes at room temperature in 50 mM potassium phosphate buffer, pH 6.5, containing 50 mM NaCl, 2 mM EDTA, and 5% ethanol. The modified antibody was purified using a column of SEPHADEX™ G25F equilibrated and eluted in 50 mM potassium phosphate buffer, pH 6.5, containing 50 mM NaCl and 2 mM EDTA. Modified antibody was conjugated with DM1 (1.7 fold molar excess over linker) dissolved in dimethylacetamide (DMA, final concentration 3%). After overnight incubation at room temperature, the conjugated antibody samples were purified by chromatography on SEPHADEX™ G25F equilibrated in phosphate buffered saline (PBS), pH 6.5.

One portion of the conjugate was held for one day at 4 ° C, diafiltered by membrane-based TFF against 10 mM sodium succinate, pH 5.5, and placed at 4 ° C. A second portion of the conjugate was held for thirty days, diafiltered by membrane-based TFF against 10 mM sodium succinate, pH 5.5, and placed at 4 °C. Free drug was measured as described in Example 1. After eight weeks of storage at 4 °C, the sample that had been diafiltered after a one day hold had 1.7% free drug, whereas the sample diafiltered after a thirty day hold had 0.8% free drug. This result demonstrates that a prolonged hold step lowers the level of free drug in the conjugate.

### EXAMPLE 4B

This example demonstrates a process for preparing a conjugate comprising an antibody chemically coupled to a drug, which process comprises a prolonged hold step prior to diafiltration by membrane-based TFF and shows that holding at a higher pH (6.5) is more beneficial.

The BIWA 4 monoclonal antibody (final concentration of 20 mg/mL) was reacted with SPP (4.4 - 4.6 fold molar excess of SPP) for approximately 120 minutes at room temperature in 50 mM potassium phosphate buffer, pH 6.5, containing 50 mM NaCl, 2 mM EDTA, and 5% ethanol. The modified antibody was purified using a column of SEPHADEX™ G25F equilibrated and eluted in 35 mM sodium citrate buffer, containing 150 mM NaCl and 2 mM EDTA, at a pH 5.0. Modified antibody was conjugated with DM1 (1.7 fold molar excess over linker) dissolved in dimethylacetamide (DMA, final concentration 3%). After overnight incubation at room temperature, the conjugated antibody samples were purified by chromatography on SEPHADEX™ G25F equilibrated in either phosphate buffered saline (PBS), pH 6.5 (Sample A) or 10 mM sodium succinate, pH 5.5 (Sample B). Both samples were held at a conjugate concentration of 2 mg/mL at 2 - 8° C for 29 days. Sample A was then diafiltered against PBS, pH 6.5. Sample B was diafiltered against 10 mM sodium succinate, pH 5.5. Both samples were stored at 2 - 8° C and analyzed for free drug at intervals as described in Example 1A. The results of this analysis are shown in Table 3.

**Table 3: Effect of Hold pH on Released Free Drug**

| | **Hold pH** | **Formulation** | **Free Drug (%) Released on Storage at 4° C** | | | |
|---|---|---|---|---|---|---|
| | | **pH** | **T = 0** | **2 wk** | **4 wk** | **6 wk** |
| Sample A | 6.5 | 6.5 | 0.4 | 0.8 | 1.0 | 1.4 |
| Sample B | 5.5 | 5.5 | 0.4 | 1.9 | 2.6 | 3.6 |

As shown by the data set forth in Table 3, stability (with respect to free drug release) of the conjugate that had been held at pH 6.5 (Sample A) was significantly greater than that of the conjugate sample (Sample B) that had been held at pH 5.5. It should be noted that the final formulation of Sample A was at pH 6.5, compared to pH 5.5 for Sample B. However, as disclosed in U.S. Patent Application Publication 2004/0241174 A1, formulating a conjugate at pH 5.5 confers significant stability (with respect to free drug release) compared to formulating at pH 6.5. These results confirm the stabilizing effect of holding the conjugate at pH 6.5, as compared to pH 5.5.

### EXAMPLE 5

This example demonstrates a process for preparing a conjugate comprising an antibody chemically coupled to a drug at pH less than 6.

The huN901 antibody was modified with SPP (molar excess of SPP as shown in Table 3) for 90-120 minutes at room temperature in 50 mM potassium phosphate buffer, pH 6.5, containing 50 mM NaCl, 2 mM EDTA, and 5% ethanol. Aliquots of modified antibody were then purified on separate columns of G25F equilibrated in either 35 mM sodium citrate buffer, pH 5.0, containing 150 mM NaCl, or 50 mM potassium phosphate buffer, pH 6.5, containing 50 mM NaCl and 2 mM EDTA. Modified antibody from each solution was conjugated with DM1 and purified on SEPHADEX™ G25 as described in Example 1. Drug/antibody ratios were also determined as described in Example 1. Linker/antibody ratios were determined by measuring releasable pyridyldithio groups as described in Example 2. The results of the analysis of the samples conjugated in the solutions with different pH values are shown in Table 4.

**Table 4: Effect of pH on Conjugate Preparation**

| **Conjugation Buffer** | **Molar Excess of SPP** | **Linker/Antibody Ratio** | **Drug/Antibody Ratio** |
|---|---|---|---|
| 35 mM sodium citrate, 150 mM NaCl, pH 5.0 | 5.7 | 4.2 | 3.6 |
| 50 mM potassium phosphate, 50 mM NaCl, 2 mM EDTA, pH 6.5 | 7.0 | 4.8 | 3.6 |

As shown by the data set forth in Table 4, when conjugation is performed at a lower pH, a smaller excess of SPP is required to achieve the same final drug/antibody level observed at higher pH. As a result, less DM1 is required to achieve the same drug/antibody level, since the amount of drug added is based on the measured linker/antibody ratio. The more efficient use of linker and drug at lower pH not only reduces material costs but also is indicative of reduced side reactions during the conjugation reaction.

### EXAMPLE 6

This example further demonstrates the beneficial effects of conjugating a modified antibody with a drug at a pH of below 6.0.

The huN901 monoclonal antibody (final concentration of 8 mg/ml) was incubated with N-succinimidyl 4-(2-pyridyldithio)pentanoate (SPP, 5.6-fold molar excess) for approximately 180 minutes at 20° C in 50 mM potassium phosphate buffer (pH 7.5) containing 50 mM NaCl, 2 mM EDTA, and 5% ethanol. In a first group, the reaction mixture was purified using a column of SEPHADEX™ G25F resin equilibrated and eluted in 50 mM sodium citrate buffer (pH 5.0) containing 50 mM NaCl and 2 mM EDTA. In a second group, the reaction mixture was purified using a column of SEPHADEX™ G25F resin equilibrated and eluted in 50 mM potassium phosphate buffer (pH 6.5) containing 50 mM NaCl and 2 mM EDTA. Both samples were conjugated with DM1 (1.7-fold molar excess over bound linker) for 3, 19, 25, 48, and 120 hours at room temperature in a final concentration of dimethylacetamide (DMA) of 3%.

Thus, the first group of samples was conjugated in 50 mM sodium citrate buffer (pH 5.0) containing 50 mM NaCl and 2 mM EDTA, and the second group of samples was conjugated in 50 mM sodium phosphate buffer (pH 6.5), containing 50 mM NaCl and 2 mM EDTA. The samples were then purified using a column of SEPHADEX™ G25F resin equilibrated and eluted in 50 mM potassium phosphate buffer (pH 6.5) containing 50 mM NaCl.

In both groups, linker/antibody ratios were determined by treatment with dithiothreitol to release pyridine-2-thione, which has an extinction coefficient of 8,080 M⁻¹cm⁻¹ at 343 nM. Drug/antibody ratios were determined spectrophotometrically (wavelengths of 280 nm and 252 nm) for the conjugation step.

The first group had a 4.3 linker/antibody ratio. The second group had a 4.2 linker/antibody ratio.

The drug/antibody ratios over time for the two groups are set forth in Table 5.

**Table 5: Rate of Incorporation of DM1 into SPP-modified huN901 as a Function of Conjugation pH**

| **Reaction Time (hours)** | **Drug/Antibody Ratio (mol/mol)** | |
|---|---|---|
| | **pH 5.0 Conjugation** | **pH 6.5 Conjugation** |
| 3 | 2.43 | 2.97 |
| 19 | 3.38 | 3.28 |
| 25 | 3.41 | Not tested (N/T) |
| 48 | 3.46 | 3.17 |
| 120 | 3.44 | 2.85 |

As is apparent from the data set forth in Table 5, conjugate that is made by conjugating the modified antibody with the drug at a pH of 5.0 reaches a higher and more stable level of bound drug during the course of the conjugation reaction than conjugate made at a conjugation pH of 6.5. In addition to increased stability, the results indicate that a higher drug/antibody level is achieved upon conjugation at pH 5.0 than when using the same amount of drug at a conjugation pH of 6.5, thereby indicating more efficient usage of drug at pH 5.0.

In both groups, the conjugate monomer amounts were determined over time. The resulting data are set forth in Table 6.

**Table 6: Effect of Conjugation pH on Level of Conjugate Monomer During Conjugation of SPP-modified huN901 with DM1**

| **Reaction Time (hours)** | **Conjugate Monomer (%)** | |
|---|---|---|
| | **pH 5.0 Conjugation** | **pH 6.5 Conjugation** |
| 3 | 98.5 | 98.0 |
| 19 | 98.8 | 98.2 |
| 25 | 99.1 | N/T |
| 48 | 99.2 | 98.3 |
| 120 | 99.2 | 97.8 |

As is apparent from the data set forth in Table 6, conjugate that is made by conjugating the modified antibody with the drug at a pH of 5.0 has a higher level of conjugate monomer than conjugate made at a conjugation pH of 6.5.

### EXAMPLE 7

This example further demonstrates a process for preparing a conjugate comprising an antibody chemically coupled to a drug at pH less than 6.

BIWA 4 antibody was modified with SPP (molar excess of SPP as shown in Table 6) for 120-140 minutes at room temperature in 50 mM potassium phosphate buffer, pH 6.5, 50 mM NaCl, 2 mM EDTA, and 5% ethanol. Aliquots of modified antibody were purified on separate NAP 25 columns equilibrated in buffers having various pH values (pH 4.6 - 6.5). The pH 4.6 - 5.9 buffers were composed of 35 mM sodium citrate, 150 mM sodium chloride, and 2 mM EDTA. The pH 6.5 buffer was PBS with 2 mM EDTA.

Modified antibody at each pH was conjugated with DM1 (1.7 fold molar excess over linker) in dimethylacetamide (DMA, final concentration 3%). After incubation for 17-18 hours at room temperature, the conjugated antibody samples were purified by chromatography on NAP 25 columns equilibrated in PBS, pH 6.5. Results of this analysis are shown in Table 7. Linker/antibody ratios (L/A in Table 7) were determined by measurement of releasable pyridyldithio groups as described in Example 2. Drug/antibody ratios (D/A in Table 7) were determined as described in Example 1A. Conjugate monomer, high molecular weight species, and low molecular weight species were determined by SEC-HPLC using a TSKG3000SWXL column equilibrated and developed in 0.2 M potassium phosphate buffer, pH 7.0, containing 0.2 M potassium chloride, and 20% isopropanol. The conjugation step yield was determined by dividing the yield of conjugated antibody (calculated as described in Example 1A) by the amount of modified antibody that was used in the conjugation step (determined spectrophotometrically at a wavelength of 280 nm).

**Table 7: Effect of pH on Conjugate Preparation**

| **Buffer** | **SPP Molar Excess** | **L/A** | **D/A** | **Monomer (%)** | **High MW (%)** | **Low MW (%)** | **Conjugation Step Yield (%)** |
|---|---|---|---|---|---|---|---|
| pH 4.6 | 4.7 | 3.8 | 3.6 | 97.5 | 2.2 | 0.4 | 74 |
| pH 5.1 | 4.4 | 4.7 | 3.6 | 97.6 | 1.9 | 0.6 | 75 |
| pH 5.6 | 5.0 | 4.9 | 3.6 | 97.7 | 1.5 | 0.8 | 85 |
| pH 5.9 | 5.5 | 5.3 | 3.7 | 96.4 | 2.3 | 1.4 | 76 |
| pH 6.5 | 6.6 | 6.4 | 3.7 | 95.1 | 2.8 | 1.9 | 71 |

The data set forth in Table 7 demonstrate that conjugation of SPP-modified BIWA 4 with DM1 was more efficient at a pH below 6.0, compared with conjugation at pH 6.5. The amounts of SPP linker and DM1 required to reach a particular final drug/antibody ratio was reduced at lower pH. In addition, levels of conjugate monomer, high molecular weight species, and low molecular weight species were more optimal, and yields are improved at lower pH.

### EXAMPLE 8

This example demonstrates a process for preparing a conjugate comprising an antibody chemically coupled to a drug, wherein the pH of the conjugation reaction is greater than 7.

The huB4 antibody was reacted with N-succinimidyl 4-(2-pyridyldithio)butanoate (SPDB) (6.0-fold molar excess) for 120 minutes at room temperature in 50 mM potassium phosphate buffer pH 6.5, 50 mM NaCl, 2 mM EDTA, and 5% ethanol. Various aliquots of modified antibody were then purified on separate NAP 25 columns equilibrated in buffers with various pH values (6.1 - 8.0). Modified antibody from solutions with these different pH values was conjugated with DM4 (1.7 fold molar excess over linker) in dimethylacetamide (DMA, final concentration 3%). After incubation for 16-18 hours at room temperature, the conjugated antibody samples were purified by chromatography on SEPHADEX™ G25F equilibrated in PBS, pH 6.5. Results of this analysis are shown in Table 8. Linker/Antibody ratios (L/A in Table 8) were determined by measuring the releasable pyridyldithio groups as described in Example 2. Drug/antibody ratios (D/A in Table 8) were determined as described in Example 1A. The conjugate monomer and the conjugation step yield were determined as described in Example 8.

**Table 8: Effect on pH on Conjugate Preparation**

| **Buffer** | **L/A** | **D/A** | **Monomer (%)** | **Conjugation Step Yield (%)** |
|---|---|---|---|---|
| PBS, pH 6.1 | 4.7 | 4.1 | 92.3 | 74 |
| PBS, pH 6.5 | 4.9 | 4.2 | 93.6 | 73 |
| PBS, pH 7.0 | 4.8 | 4.3 | 93.0 | 80 |
| PBS, pH 7.5 | 4.7 | 4.3 | 94.2 | 82 |
| PBS, pH 8.0 | 4.7 | 4.4 | 94.8 | 83 |

The data set forth in Table 8 demonstrate that at a pH of 7.0 and higher, conjugate yields and conjugate monomer levels were more optimal than at pH 6.1 - 6.5. In addition, a higher drug/antibody ratio was achieved for the same amount of DM4 utilized, which indicates a more efficient incorporation of DM4 at higher pH.

### EXAMPLE 9

This example demonstrates the beneficial effects of conjugating a modified antibody with a drug at a pH of above 6.5.

In a first experiment, CNTO95 antibody (final concentration of 10 mg/ml) was modified with N-succinimidyl 4-(2-pyridyldithio)butanoate (SPDB, 4.5 fold molar excess) for 120 minutes at 20° C in 10 mM sodium phosphate buffer (pH 7.5) containing 2.7 % sucrose and 5% ethanol. The reaction mixture was purified using a column of SEPHADEX™ G25F resin equilibrated and eluted in 12.5 mM potassium phosphate buffer, pH 6.5, 12.5 mM NaCl, and 0.5 mM EDTA. The purified modified antibody was then divided into two groups. In the first group, conjugation was performed in 12.5 mM potassium phosphate at pH 6.5 containing 12.5 mM NaCl, 0.5 mM EDTA, 3% DMA, and 1.7 fold molar excess drug per linker at 20° C. In the second group, the conjugation was performed in the buffer with the adjusted pH of 7.5. The conjugated antibody was purified over NAP-10 columns equilibrated in 10 mM sodium citrate buffer, pH 5.5, containing 135 mM NaCl.

The drug/antibody ratio was measured for both groups. The resulting data are set forth in Table 9.

**Table 9: Drug/Antibody Ratio at Conjugation Reaction of pH 6.5 versus 7.5**

| **Reaction Time (hours)** | **Drug/Antibody Ratio at Conjugation Reaction pH 6.5** | **Drug/Antibody Ratio at Conjugation Reaction pH 7.5** |
|---|---|---|
| 0.5 | N/T | 3.0 |
| 1 | 2.3 | 3.4 |
| 1.5 | N/X | 3.5 |
| 2 | 2.8 | 3.5 |
| 2.75 | N/T | 3.6 |
| 3.5 | 3.2 | 3.6 |
| 5 | 3.4 | 3.7 |

As shown by the data set forth in Table 9, conjugation proceeds faster at pH 7.5 than at pH 6.5.

In a second experiment, huB4 humanized monoclonal antibody was modified with either (a) a 4.9-fold molar excess of SPDB relative to antibody, or (b) a 4.8-fold molar excess of SPDB relative to antibody. In both situations, reaction was in 50 mM potassium phosphate, 50 mM potassium chloride, and 2 mM EDTA (pH 6.5) in 5% ethanol for a total of 120 minutes at room temperature. Sample (a) was purified over a column of SEPHADEX™ G25F resin equilibrated in 50 mM potassium phosphate, 50 mM sodium chloride, and 2 mM EDTA at pH 6.5. Sample (b) was purified equivalently except that the chromatography buffer was adjusted to pH 7.5. Both samples were conjugated with DM4 (1.7 fold molar excess over bound linker) for 18 hours at room temperature in a final concentration of dimethylacetamide (DMA) of 3%.

Thus, sample (a) was conjugated at pH 6.5, and sample (b) was conjugated at pH 7.5. The samples were then purified over a column of SEPHADEX™ G25F resin equilibrated in 9.6 mM potassium phosphate and 4.2 mM sodium chloride at pH 6.5. Both samples were incubated at 4° C for up to 7 months and subjected to analysis of released free drug at intervals. The resulting data are set forth in Table 10.

**Table 10: Release of Free Drug Over Time from Samples Conjugated at pH 6.5 and 7.5**

| **Time (months)** | **pH 6.5 Conjugation** | **pH 7.5 Conjugation** |
|---|---|---|
| 0 | 1.0 | 0.8 |
| 1.5 | 1.8 | 1.0 |
| 2.5 | 3.2 | 1.9 |
| 7 | 4.0 | 2.8 |

As shown by the data set forth in Table 10, release of free drug is substantially slower from sample (b) that had been conjugated at pH 7.5 relative to sample (a) that had been conjugated at pH 6.5. Accordingly, drug conjugate product prepared at pH 7.5 is shown to be more stable with respect to release of free drug over time as compared to the drug conjugate product prepared at pH 6.5. The conjugation at pH 7.5 also shows a better drug incorporation than at pH 6.5, thereby requiring less drug to be used.

### EXAMPLE 10

This example demonstrates a process for preparing a conjugate comprising an antibody chemically coupled to a drug, wherein the conjugation reaction mixture contains sucrose.

The J591 antibody was modified with SPP (7.0-fold molar excess of SPP over antibody) for 130 minutes at room temperature in 50 mM potassium phosphate buffer, pH 6.0, containing 2 mM EDTA and 5% ethanol. Modified antibody was then purified by chromatography on SEPHADEX™ G25F equilibrated in 50 mM potassium phosphate buffer, pH 6.0, containing 2 mM EDTA. Modified antibody was conjugated with DM1 (1.7 fold molar excess over linker) in dimethylacetamide (DMA, final concentration 3%), in either the presence or absence of 10% (w/v) sucrose. After incubation overnight at room temperature, the samples conjugated without sucrose were purified by chromatography on SEPHADEX™ G25F equilibrated in 20 mM sodium succinate buffer, pH 5.5. The samples conjugated in the presence of sucrose were purified by chromatography on SEPHADEX™ G25F equilibrated with 20 mM sodium succinate buffer, pH 5.5, containing 5% (w/v) sucrose. The conjugation step yield (determined as described in Example 7) for the process run with added sucrose was 62% (average of two runs), whereas the yield for the process without sucrose was 49% (average of two runs). Thus, the results of this example demonstrate that sucrose improves the yield of the conjugation reaction.

### EXAMPLE 11

This example demonstrates a process for preparing a conjugate comprising an antibody chemically coupled to a drug, wherein the conjugation reaction mixture contains sucrose.

The BIWA 4 antibody was modified with SPP (6.6-fold molar excess of SPP over antibody) for 120 minutes at room temperature in 50 mM potassium phosphate buffer, pH 6.5, containing 50 mM NaCl, 2 mM EDTA, and 5% ethanol. Modified antibody was purified by chromatography on SEPHADEX™ G25F equilibrated in 50 mM potassium phosphate buffer pH 6.5, containing 50 mM NaCl and 2 mM EDTA. Purified modified antibody was then conjugated with DM1 (1.7 fold molar excess over linker) in dimethylacetamide (DMA, final concentration 3%), in either the presence or absence of 10% (w/v) sucrose. After incubation overnight at room temperature, the sample conjugated without sucrose was purified by chromatography on SEPHADEX™ G25F equilibrated in PBS, pH 6.5. The sample conjugated in the presence of sucrose was divided into two portions. One portion was purified by chromatography on SEPHADEX™ G25F equilibrated in PBS, pH 6.5. The second portion was purified by chromatography on SEPHADEX™ G25F equilibrated in PBS, pH 6.5 containing 10% (w/v) sucrose. The conjugation step yield (determined as described in Example 7) for the process run with sucrose in both the conjugation reaction and the subsequent G25 column was 80%, whereas the yield for the process with sucrose only in the conjugation reaction was 68%, and the yield for the process run without sucrose was 62%. Thus, the results of this example demonstrate that sucrose improves the yield of the conjugation reaction and subsequent purification step.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

The present invention further comprises, inter alia, the following embodiments:
1. A process for preparing a conjugate comprising a cell binding agent chemically coupled to a drug, which process comprises:
   (a) contacting a cell binding agent with a bifunctional crosslinking reagent to covalently attach a linker to the cell binding agent and thereby prepare a first mixture comprising cell binding agents having linkers stably and unstably bound thereto,
   (b) subjecting the first mixture to non-adsorptive chromatography to purify the cell binding agent having linkers bound thereto from other components of the first mixture and thereby prepare a purified first mixture,
   (c) conjugating a drug to the cell binding agents having linkers bound thereto by reacting the cell binding agents having linkers bound thereto with a drug in a solution having a pH of about 4.5 to about 8 to prepare a second mixture comprising (i) cell binding agent chemically coupled through the linker to the drug, (ii) free drug, and (iii) solvents and reaction by products.
   (d) subjecting the second mixture to non-adsorptive chromatography to purify the cell binding agents chemically coupled through the linkers to the drug from the other components of the second mixture and thereby prepare a purified second mixture,
   (e) optionally subjecting the purified second mixture to tangential flow filtration (TFF) to isolate a conjugate comprising the cell binding agent chemically coupled to the drug, and
   (f) holding the mixture between at least one of steps a-b, steps b-c, steps c-d, and steps d-e to release the unstably bound linkers from the cell binding agent
2. The process of claim 1, wherein the mixture is held between steps a-b.
3. The process of claim 1, wherein the mixture is held between steps b-c.
4. The process of claim 1, wherein the mixture is held between steps c-d.
5. The process of claim 1, wherein the mixture is held between steps d-e.
6. The process of any of claims 1-5, wherein the cell binding agent is selected from the group consisting of interferons, interleukin 2 (IL-2), interleukin 3 (IL-3), interleukin 4 (IL-4), interleukin 6 (IL-6), insulin, EGF, TGF-α, FGF, G-CSF, VEGF, MCSF, GM-CSF, transferrin, and antibodies.
7. The process of claim 6, wherein the cell binding agent is an antibody.
8. The process of claim 7, wherein the antibody is a monoclonal antibody.
9. The process of claim 8, wherein the antibody is a humanized monoclonal antibody.
10. The process of claim 7, wherein the antibody is selected from the group consisting of huN901, huMy9-6, huB4, huC242, trastuzumab, bivatuzumab, sibrotuzumab, CNTO95 and huDS6 and rituximab.
11. The process of any of claims 1-10, wherein the drug is a cytotoxic agent.
12. The process of claim 11, wherein the cytotoxic agent is selected from the group consisting of maytansinoids, taxanes, CC1065, and analogs of the foregoing.
13. The process of claim 12, wherein the drug is a maytansinoid.
14. The process of claim 13, wherein the maytansinoid comprises a thiol group.
15. The process of claim 14, wherein the maytansinoid is DM1.
16. The process of claim 14, wherein the maytansinoid is DM4.
17. The process of any of claims 1-16, wherein the cell binding agent is chemically coupled to the drug via chemical bonds selected from the group consisting of disulfide bonds, acid labile bonds, photolabile bonds, peptidase labile bonds, thioether bonds, and esterase labile bonds.
18. The process of any of claims 1-17, wherein the solution in step (c) and/or (d) further comprises sucrose.
19. The process of any of claims 1-18, wherein the solution in step (c) and/or (d) further comprises a buffering agent selected from the group consisting of a citrate buffer, an acetate buffer, a succinate buffer, and a phosphate buffer.
20. The process of any of claims 1-19, wherein unstably bound linkers are released from the cell binding agent by adjusting the pH of the mixture of step (f).
21. The process of any of claims 1-20, wherein the holding step comprises incubating the mixture at 4° C at a pH of about 6-7.5 for about 12 hours to about 4 weeks.
22. The process of any of claims 1-20, wherein the holding step comprises incubating the mixture at 25° C at a pH of about 6-7.5 for about 12 hours to about 1 week.
23. The process of any of claims 1-20, wherein the holding step comprises incubating the mixture at 4° C at a pH of about 4.5-5.9 for about 5 hours to about 5 day.
24. The process of any of claims 1-20, wherein the holding step comprises incubating the mixture at 25° C at a pH of about 4.5-5.9 for about 5 hours to about 1 day.
25. The process of any of claims 1-24, wherein the holding step further comprises addition of a nucleophile to the mixture.
26. The process of claim 25, wherein the nucleophile is a primary nucleophilic amine, a secondary nucleophilic amine, or a combination thereof.
27. The process of claim 26, wherein the nucleophile is selected from the group consisting of glycylglycine, taurine, diethanolamine, lysine, hydroxylamine, imidazole, ethylamine, 4-amino-1-butanol, polylysine, lysine-bearing peptides, poly(ethyleneimine), hydrazine, glycine, ethanolamine, 2-amino-2-(hydrox-ymethyl)-1,3-propane-diol and combinations thereof.
28. The process of any of claims 1-27, wherein the non-adsorptive chromatography is selected from the group consisting of SEPHADEX™ resins, SEPHACRYL™ resins, SUPERDEX™ resins, and BIO-GEL^{®} resins.
29. A process for preparing a conjugate comprising a cell binding agent chemically coupled to a drug, which process comprises:
   (a) contacting a cell binding agent with a drug bearing an active ester and thereby prepare a mixture comprising cell binding agents having drugs stably and unstably bound thereto,
   (b) subjecting the mixture to non-adsorptive chromatography to purify the cell binding agent having drug bound thereto from other components of the mixture and thereby prepare a purified mixture,
   (c) optionally subjecting the purified mixture to tangential flow filtration (TFF) to isolate a conjugate comprising the cell binding agent chemically coupled to the drug, and
   (d) holding the mixture between at least one of steps a-b and b-c to release the unstably bound drugs from the cell binding agent.
30. The process of claim 29, wherein the mixture is held between steps a-b.
31. The process of claim 29, wherein the mixture is held between steps b-c.
32. The process of any of claims 29-31, wherein the cell binding agent is selected from the group consisting of interferons, interleukin 2 (IL-2), interleukin 3 (IL-3), interleukin 4 (IL-4), interleukin 6 (IL-6), insulin, EGF, TGF-α, FGF, G-CSF, VEGF, MCSF, GM-CSF, transferrin, and antibodies.
33. The process of claim 32, wherein the cell binding agent is an antibody.
34. The process of claim 33, wherein the antibody is a monoclonal antibody.
35. The process of claim 34, wherein the antibody is a humanized monoclonal antibody.
36. The process of claim 34, wherein the antibody is selected from the group consisting of huN901, huMy9-6, huB4, huC242, CNTO95, huDS6, trastuzumab, bivatuzumab, sibrotuzumab, and rituximab.
37. The process of any of claims 29-37, wherein the drug is a cytotoxic agent.
38. The process of claim 37, wherein the cytotoxic agent is selected from the group consisting of maytansinoids, taxanes, CC1065, and analogs of the foregoing.
39. The process of claim 38, wherein the drug is a maytansinoid.
40. The process of claim 39, wherein the maytansinoid comprises a thiol group.
41. The process of claim 40, wherein the maytansinoid is DM1.
42. The process of claim 40, wherein the maytansinoid is DM4.
43. The process of any of claims 29-42, wherein the cell binding agent is chemically coupled to the drug via chemical bonds selected from the group consisting of disulfide bonds, acid labile bonds, photolabile bonds, peptidase labile bonds, thioether bonds, and esterase labile bonds.
44. The process of any of claims 29-43, wherein the solution in step (a) and/or (b) further comprises sucrose.
45. The process of any of claims 29-43, wherein the solution in step (a) and/or (b) further comprises a buffering agent selected from the group consisting of a citrate buffer, an acetate buffer, a succinate buffer, and a phosphate buffer.
46. The process of any of claims 29-45, wherein unstably bound linkers are released from the cell binding agent by adjusting the pH of the mixture of step (d).
47. The process of any of claims 29-46, wherein the holding step comprises incubating the mixture at 4° C at a pH of about 6-7.5 for about 12 hours to about 4 weeks.
48. The process of any of claims 29-46, wherein the holding step comprises incubating the mixture at 25° C at a pH of about 6-7.5 for about 12 hours to about 1 week.
49. The process of any of claims 29-48, wherein the holding step further comprises addition of a nucleophile to the mixture.
50. The process of claim 49, wherein the nucleophile is a primary nucleophilic amine, a secondary nucleophilic amine, or a combination thereof.
51. The process of claim 50, wherein the nucleophile is selected from the group consisting of glycylglycine, taurine, diethanolamine, lysine, hydroxylamine, imidazole, ethylamine, 4-amino-1-butanol, polylysine, lysine-bearing peptides, poly(ethyleneimine), hydrazine, glycine, ethanolamine, 2-amino-2-(hydroxymethyl)-1,3-propane-diol and combinations thereof.
52. The process of any of claims 29-51, wherein the non-adsorptive chromatography is selected from the group consisting of SEPHADEX™ resins, SEPHACRYL™ resins, SUPERDEX™ resins, and BIO-GEL^{®} resins.

## Claims

1. A process for preparing a conjugate comprising a cell binding agent chemically coupled to a cytotoxic agent, which process comprises:
(a) contacting a cell binding agent with a cytotoxic agent bearing an active ester and thereby prepare a mixture comprising cell binding agents having cytotoxic agents stably and unstably bound thereto,
(b) subjecting the mixture to non-adsorptive chromatography to purify the cell binding agent having cytotoxic agent bound thereto from other components of the mixture and thereby prepare a purified mixture,
(c) optionally subjecting the purified mixture to tangential flow filtration (TFF) to isolate a conjugate comprising the cell binding agent chemically coupled to the cytotoxic agent, and
(d) holding the mixture between at least one of steps a-b and b-c to release unstably bound cytotoxic agents from the cell binding agent.

2. A process for preparing a conjugate comprising a cell binding agent chemically coupled to a cytotoxic agent, which process comprises:
(a) contacting a cell binding agent with a bifunctional crosslinking reagent to covalently attach a linker to the cell binding agent and thereby prepare a first mixture comprising cell binding agents having linkers stably and unstably bound thereto,
(b) subjecting the first mixture to non-adsorptive chromatography to purify the cell binding agent having linkers bound thereto from other components of the first mixture and thereby prepare a purified first mixture,
(c) conjugating a cytotoxic agent to the cell binding agents having linkers bound thereto by reacting the cell binding agents having linkers bound thereto with a cytotoxic agent in a solution having a pH of about 4.5 to about 8 to prepare a second mixture comprising (i) cell binding agent chemically coupled through the linker to the cytotoxic agent, (ii) free cytotoxic agent, and (iii) solvents and reaction by products.
(d) subjecting the second mixture to non-adsorptive chromatography to purify the cell binding agents chemically coupled through the linkers to the cytotoxic agent from the other components of the second mixture and thereby prepare a purified second mixture,
(e) optionally subjecting the purified second mixture to tangential flow filtration (TFF) to isolate a conjugate comprising the cell binding agent chemically coupled to the cytotoxic agent, and
(f) holding the mixture between at least one of steps a-b, steps b-c, steps c-d, and steps d-e to release unstably bound linkers from the cell binding agent.

3. The process of claim 1 or 2, wherein the cell binding agent is selected from the group consisting of interferons, interleukin 2 (IL-2), interleukin 3 (IL-3), interleukin 4 (IL-4), interleukin 6 (IL-6), insulin, EGF, TGF-α, FGF, G-CSF, VEGF, MCSF, GM-CSF, transferrin, and antibodies.

4. The process of claim 3, wherein the cell binding agent is an antibody.

5. The process of claim 4, wherein the antibody is selected from the group consisting of huN901, huMy9-6, huB4, huC242, trastuzumab, bivatuzumab, sibrotuzumab, CNTO95, huDS6, and rituximab.

6. The process of any one of claims 1-5, wherein the cytotoxic agent is selected from the group consisting of maytansinoids, taxanes, CC1065, and analogs of the foregoing.

7. The process of claim 6, wherein the cytotoxic agent is a maytansinoid.

8. The process of claim 7, wherein the maytansinoid comprises a thiol group.

9. The process of claim 8, wherein the maytansinoid is N2ʹ-deacetyl-N2ʹ-(3-mercapto-1-oxopropyl)-maytansine (DM1) or N2ʹ-deacetyl-N2ʹ-(4-methyl-4-mercapto-1-oxopentyl)-maytansine (DM4).

10. The process of any one of claims 1-9, wherein the cell binding agent is chemically coupled to the cytotoxic agent via chemical bonds selected from the group consisting of disulfide bonds, acid labile bonds, photolabile bonds, peptidase labile bonds, thioether bonds, and esterase labile bonds.

11. The process of any one of claims 1-10, wherein unstably bound linkers are released from the cell binding agent by adjusting the pH of the mixture.

12. The process of any one of claims 1-11, wherein the holding step comprises:
(a) incubating the mixture at about 2° C to about 8° C for about 5 hours to 30 days,
(b) incubating the mixture at 25° C at a pH of about 6-7.5 for about 12 hours to about 1 week,
(c) incubating the mixture at between 20-30° C at pH 6.5 for about 12 hours to about 1 week,
(d) incubating the mixture at 25° C at a pH of about 4.5-5.9 for about 5 hours to about 1 day, or
(e) incubating the mixture between 20-30° C at a pH of about 5 for about 5 hours to about 3 days.
(f) incubating the mixture at 4º C at a pH of about 6-7.5 for about 12 hours to about 4 weeks; or
(g) incubating the mixture at 4° C at a pH of about 4.5-5.9 for about 5 hours to about 5 day.

13. The process of any one of claims 1-12, wherein the holding step further comprises addition of a nucleophile to the mixture.

14. The process of claim 13, wherein the nucleophile is a primary nucleophilic amine, a secondary nucleophilic amine, or a combination thereof.

15. The process of claim 14, wherein the nucleophile is selected from the group consisting of glycylglycine, taurine, diethanolamine, lysine, hydroxylamine, imidazole, ethylamine, 4-amino-1-butanol, polylysine, lysine-bearing peptides, poly(ethyleneimine), hydrazine, glycine, ethanolamine, 2-amino-2-(hydroxymethyl)-1,3-propane-diol, and combinations thereof.
